# FASCICULE DE BREVET EUROPEEN

(11) **EP 2 260 287 B1**
(45) Date de publication et mention de la délivrance du brevet: **19.06.2013**
(21) Numéro de dépôt: 09725264.7
(22) Date de dépôt: 12.03.2009
(51) Int. Cl.: G01N 1/40, G01N 1/28, G01N 33/24

(54) **DISPOSITIF DE PREPARATION D'ECHANTILLON**
VORRICHTUNG ZUR VORBEREITUNG EINER PROBE
DEVICE FOR PREPARING A SAMPLE

(30) Priorité: 12.03.2008 FR 0851612
(43) Date de publication de la demande: 15.12.2010
(73) Titulaire: Centre National d'Etudes Spatiales (C.N.E.S.), 75001 Paris (FR)
(72) Inventeur: STERNEBERG, Robert, F-94320 Thiais (FR); BUCH, Arnaud, F-75019 Paris (FR); CORREIA, Jean-Jacques, F-91120 Palaiseau (FR); CHAZALNOEL, Pascale, F-31280 Drémil-Lafage (FR)
(74) Mandataire: Novagraaf Technologies
(86) Numéro de dépôt international: PCT/FR2009/000261
(87) Numéro de publication internationale: WO 2009/118489

(56) Documents cités:
- US-A- 5 970 804
- MEUNIER D ET AL: "A laboratory pilot for in situ analysis of refractory organic matter in Martian soil by gas chromatography-mass spectrometry" ADVANCES IN SPACE RESEARCH - LABORATORY SIMULATION OF SOLAR SYSTEM PROCESSES AND EXPLORATION OF SMALL SOLAR SYSTEM OBJECTS 2007 ELSEVIER LTD; PERGAMON; THE BOULEVARD GB, vol. 39, no. 3, 2007, pages 337-344, XP0022059500 cité dans la demande
- PAUL MAHAFFY: "Exploration of the Habitability of Mars: Development of Analytical Protocols for Measurement of Organic Carbon on the 2009 Mars Science Laboratory" SPACE SCIENCE REVIEWS, KLUWER ACADEMIC PUBLISHERS, DO, vol. 135, no. 1-4, 27 juillet 2007 (2007-07-27), pages 255-268, XP019615167 ISSN: 1572-9672
- MAHONE W C: "CONCENTRATING GASEOUS CONTAMINANTS FOR MONITORING" NTIS TECH NOTES, US DEPARTMENT OF COMMERCE. SPRINGFIELD, VA, US, 1 janvier 1991 (1991-01-01), page 102, XP000383537 ISSN: 0889-8464

## Description

### Domaine technique

La présente invention se rapporte à un dispositif de préparation d'échantillon permettant de préparer un échantillon ainsi qu'un système d'analyse *in situ* d'échantillon équipé d'un tel dispositif.

Le dispositif de la présente invention permet notamment d'extraire, de fonctionnaliser, de volatiliser et de transférer les molécules organiques contenues dans un échantillon afin de les analyser.

Le dispositif de la présente invention est utilisable pour l'analyse de sol dans des environnements terrestres ou extraterrestres. Le dispositif de la présente invention peut également être utilisé dans d'autres domaines, tels que l'industrie alimentaire, l'industrie agronomique, l'industrie du parfum ou autres.

Dans la description ci-dessous, les références entre parenthèses (ref x) renvoient à la liste des références présentée après les exemples.

### Etat de la technique

L'analyse de sols et l'étude de leurs propriétés physiques, chimiques et biologiques a un intérêt dans de multiples domaines, par exemple dans le domaine de l'agriculture, de l'environnement ou de l'agroalimentaire. L'analyse de sols permet par exemple de mesurer la contamination ou la pollution de sols, d'évaluer l'impact de pratiques culturales, d'étudier le rôle des matières organiques, la fertilité ou le fonctionnement d'un sol, etc. En outre, ces informations peuvent permettre de préserver les ressources sols, de favoriser l'entretien ou la restauration de fonctions essentielles du sol, etc.

L'analyse de sols présente également un intérêt croissant dans le domaine de l'exobiologie. L'exobiologie est un domaine pluridisciplinaire qui inclut l'étude de l'origine, de l'évolution et de la distribution de la vie dans l'univers, y compris la Terre, la recherche de traces d'activités biologiques et l'étude de la chimie organique dans les milieux extraterrestres.

L'analyse de molécules organiques présentes dans un échantillon solide, par exemple un échantillon de sol, nécessite des techniques qui permettent d'extraire, de séparer, d'identifier et éventuellement de quantifier les espèces moléculaires organiques et inorganiques présentes dans les échantillons prélevés. Ainsi, l'analyse de ces molécules nécessite généralement des techniques séparatives telles que la chromatographie en phase gazeuse (CPG) couplée ou non à la spectrométrie de masse (SM).

Cependant, l'analyse de molécules présentes dans un échantillon nécessite de préparer préalablement l'échantillon et de le traiter. Cette préparation consiste notamment à extraire ces molécules de l'échantillon et à les transformer chimiquement et/ou physiquement, si nécessaire, afin de pouvoir les détecter et les analyser.

L'extraction de ces molécules nécessite des appareils et des conditions particulières qui doivent permettre de détacher efficacement les molécules organiques de la matrice qui les contient sans les dégrader. En effet, certaines molécules sont difficiles à extraire et sont fortement liées au solide, de sorte que l'extraction de ces molécules nécessite généralement des conditions dures qui entraînent souvent la dégradation de ces molécules.

On peut citer par exemple un appareil de soxhlet permettant de réaliser des extractions solide-liquide au moyen d'un solvant approprié. Mais il nécessite des temps d'extraction longs à températures élevées, ce qui peut entraîner la dégradation de certaines molécules. De plus, le dispositif est coûteux et encombrant. On peut également citer le dispositif d'extraction en phase supercritique décrit dans le document de Dadka A. et al., Journal of Hazardous materials, 2002, 93 (3), page 307-320 **(ref 1).** Cependant, l'extraction nécessite de manipuler des fluides pressurisés et le dispositif est coûteux et encombrant.

Ainsi. l'extraction, parfois difficile à mettre en oeuvre, nécessite des dispositifs et appareillages encombrants. Elle ne peut donc pas être réalisée *in situ* sur le site de prélèvement de l'échantillon.

De plus, les méthodes d'extraction existantes sont généralement utilisées pour des quantités relativement importantes d'échantillons et ne sont pas adaptées, notamment par leur encombrement, ni optimisées en termes de rendement pour des échantillons de faibles masses comme par exemple ceux utilisés pour l'analyse et la détection de molécules organiques dans des échantillons solides.

Par ailleurs, une fois les molécules extraites, elles doivent généralement être transformées de façon à être détectables et analysables par les instruments d'analyse couramment utilisés. La CPG nécessite notamment que les molécules à analyser soient volatilisées.

Ainsi, de nombreux dispositifs utilisent la pyrolyse pour transformer chimiquement et/ou physiquement les molécules organiques susceptibles d'être présentes dans un échantillon. Cette méthode conduit à la décomposition des molécules organiques en fragments généralement plus volatils.

A titre d'exemple, dans le cadre de l'exploration de la planète Mars, les deux sondes Viking ont permis d'effectuer des analyses chimiques in situ d'échantillons prélevés à la surface de Mars en utilisant à la fois un CPG classique (détecteur catharométrique) et un CPG-SM associés à des procédés de transformation chimique complexes destinés à détecter la matière organique et la présence d'une activité biologique. par pyrolyse. Cependant, les sondes Viking n'ont pas permis de détecter des traces de matière organique à la surface de Mars. En effet, les instruments de CPG-SM de Viking ne pouvaient détecter que les composés gazeux ou vaporisables (par chauffage modéré) et par conséquent ne pouvaient pas accéder aux molécules non-volatiles.

D'autres dispositifs sont connus. Par exemple le document Mowry Curtis et al., Proceeding of SPIE (2001), Vol. 4575, pages 83-90 (ref 2), décrit un dispositif permettant la détection rapide et l'identification de bactéries. Il est conçu pour analyser de la matière humide (échantillon bactérien) et volatiliser des acides gras. Ce dispositif comprend un micro-pyrolyseur constitué d'un substrat sur wafer de silicium comportant une membrane de nitrure de silicium circulaire chauffée par un élément résistif de platine. Le pyrolyseur est couplé à un spectromètre de masse au moyen d'un capillaire direct.

Ainsi, la pyrolyse est une transformation chimique qui permet en effet l'obtention de composés (fragments) volatils, Cependant, cette transformation engendre la dégradation des molécules et conduit à la dénaturation de certaines propriétés physiques et chimiques des molécules cibles comme par exemple la chiralité ou l'asymétrie. Par conséquent elle pose des problèmes pour l'identification des molécules initiales à partir de leurs nombreux fragments de pyrolyse.

Le document US 5,970,804 décrit une méthode et un appareil pour déterminer la présence ou l'absence de composé spécifique présent dans un mélange. Cet appareil comprend un réacteur étanche comprenant un moyen d'introduction de l'éehantillon, au moins une entrée et au moins une sortie étanches connectées audit réacteur et permettant la circulation d'un fluide de balayage au travers dudit réacteur, un moyen de chauffage dudit réacteur un moyen de refroidissement dudit réacteur et un moyen de commande et de contrôle de la température dans le réacteur.

Par ailleurs, la préparation d'échantillon en vue de l'analyse des molécules qu'il contient s'effectue généralement en plusieurs étapes et utilise plusieurs réacteurs de préparation. On peut par exemple citer les documents Buch et al., Planetary and Space Science, Special Astrobiology Issue, 54, 15, (2006), 1592-1599 (ref 3) et Buch et al., Journal of Chromatography A. 999, (2003), 165-174 (ref 4). Ainsi, la préparation d'échantillon nécessite des appareillages et dispositifs très encombrants et difficilement transportables sur un site de mesure.

Il existe donc un réel besoin de disposer d'un dispositif de préparation d'échantillon en vue de l'analyse chimique *in situ* des molécules qu'il contient, palliant ces défauts, inconvénients et obstacles de l'art antérieur. En particulier, il existe un réel besoin de disposer d'un dispositif compatible avec les contraintes d'encombrement et les contraintes spatiales, permettant de traiter et préparer *in situ* les échantillons prélevés de façon rapide, quantitative, sans dénaturation des composés et en un minimum d'étapes. En outre, il existe un réel besoin de disposer d'un dispositif de préparation d'échantillon capable d'être couplé à un instrument d'analyse et permettant d'optimiser la sensibilité, la qualité et l'interprétation des analyses.

### Description de l'invention

La présente invention a précisément pour but de répondre à ces besoins et inconvénients de l'art antérieur en proposant un dispositif de préparation d'échantillon, transportable sur un site de mesure, permettant une transformation de l'échantillon visant à permettre son analyse complète ainsi que le transfert des molécules organiques à analyser vers un instrument d'analyse.

A cet effet, et selon un premier aspect, l'invention propose un Dispositif de Préparation d'Echantillon (DPE) comprenant :
- un réacteur étanche comportant un moyen d'introduction d'un échantillon comprenant des molécules organiques à analyser ;
- au moins une entrée et une sortie étanches connectées audit réacteur et permettant la circulation d'un fluide de balayage au travers dudit réacteur ;
- un moyen de chauffage dudit réacteur à une vitesse moyenne de 10 à 50 °C/seconde, par exemple de 25 à 50 °C/seconde ;
- un moyen de refroidissement dudit réacteur à une vitesse moyenne de 10 à 50 °C/seconde, par exemple de 25 à 50 °C/seconde, ledit le moyen **de refroidissement étant une plaque de refroidissement traversée par un circuit de refroidissement, ledit moyen de refroidissement étant mis en contact avec le réacteur au moyen d'une presse ;**
- un moyen de commande et de contrôle de température dans le réacteur ; et
- un moyen de contrôle de la pression dans le réacteur ; et
- **une sonde appliquant des ultrasons à l'intérieur du réacteur.**

On entend par « échantillon » au sens de la présente invention tout échantillon solide, liquide ou gazeux, de préférence solide, susceptible de contenir des molécules organiques. Il peut s'agir par exemple d'échantillons de sol prélevé en surface ou en profondeur, provenant par exemple d'un grattage ou d'une carotte de sol: il peut s'agir par exemple de différents types de sols, par exemple roche, gravas, pierres, sable, poussière, etc. d'origine terrestre ou extraterrestre. Il peut provenir par exemple d'une autre planète que la Terre, d'un satellite naturel ou d'une météorite. Il peut s'agir par exemple d'un sol provenant de Mars, la Lune, Titan, etc.

Au sens de la présente invention, l'échantillon peut comporter des molécules organiques, qui peuvent être volatiles ou non-volatiles, présentes ou non à l'état de traces. Il peut s'agir de molécules chimiques ou de molécules biologiques, comme par exemple des acides carboxyliques, des acides aminés, des peptides, des protéines, des sucres, des acides gras, des lipides, des bases puriques, des amines, des hydrocarbures aromatiques polycycliques (HAP), des acides et bases nucléiques, de l'ADN ou de fragments de ces molécules. On entend par « molécules organiques non-volatiles » au sens de la présente invention toute molécule organique qui ne passe pas à l'état gazeux par chauffage sans dégradation, c'est-à-dire dont la volatilisation entraîne la dégradation. Il peut par exemple s'agir de molécules organiques de la liste précitée.

Le DPE de la présente invention constitue donc un système de préparation d'échantillon c'est-à-dire de traitement d'échantillon permettant notamment l'extraction, la fonctionnalisation et la volatilisation de molécules organiques présentes dans un échantillon ainsi que le transfert de ces molécules vers un instrument d'analyse. En d'autres termes, le DPE de l'invention permet de détacher des molécules organiques, et notamment des molécules organiques non-volatiles, contenues dans un échantillon sans les détruire, de les fonctionnaliser afin de les rendre plus volatiles, de les volatiliser et de les transférer en vue de leur analyse. Ainsi, le DPE de la présente invention peut être destiné à l'analyse chimique *in situ* d'échantillons. Les moyens d'analyse et les instruments d'analyse utilisables dans le cadre de l'invention sont décrits ci-dessous.

En outre, le DPE de la présente invention permet de traiter (c'est-à-dire d'extraire, de fonctionnaliser et de volatiliser) les molécules organiques de l'échantillon, qualitativement et quantitativement. Le DPE de la présente invention permet également de transférer qualitativement et quantitativement les molécules organiques extraites, fonctionnalisées et volatilisées vers un instrument d'analyse, grâce à l'entrée et la sortie d'un fluide.

Par « qualitativement », on entend au sens de la présente invention de façon à préserver la nature et qualité des échantillons, c'est-à dire de façon à limiter voir éviter la dégradation des molécules organiques lors des étapes d'extraction, de fonctionnalisation et de volatilisation, notamment lors de l'étape d'extraction. Par « quantitativement », on entend au sens de la présente invention de façon à traiter (c'est-à-dire extraire, fonctionnaliser et volatiliser) le plus possible de molécules organiques présentes dans l'échantillon. La présente invention permet avantageusement, par exemple en utilisant un procédé de "fonctionnalisation extractive" approprié, d'atteindre un bon rendement de récupération des molécules organiques, notamment supérieur à 80%, et donc d'obtenir une quantité suffisante de molécules organiques pour les analyser.

Par « moyen d'introduction », on entend au sens de la présente invention un passage de l'extérieur vers l'intérieur du réacteur, et inversement. Il peut s'agir par exemple d'une ouverture avec un couvercle, d'un sas, d'une canalisation munie d'une vanne, d'un moyen d'injection par vis, d'un moyen d'injection par piston, etc. L'ouverture et la fermeture de ce moyen d'introduction peuvent être effectuées manuellement ou automatiquement, par exemple commandée par une unité de commande. Lorsque le moyen d'introduction est fermé, le réacteur est étanche. Le réacteur peut être par exemple constitué d'un contenant et de son couvercle. Le couvercle est tel qu'il peut fermer le réacteur de manière étanche, par exemple au moyen d'un pas de vis et d'un joint torique. Ainsi, afin d'assurer l'étanchéité du réacteur, un joint d'étanchéité peut être est disposé entre le contenant et son couvercle. Le joint est de préférence réalisé en un matériau étanche résistant à des températures supérieures à 500 °C. À titre d'exemple, le joint d'étanchéité peut notamment être réalisé en graphite, en inox et tout autre métal résistant à des températures de 500 °C.

L'entrée et la sortie étanches connectées audit réacteur permettent la circulation d'un fluide de balayage au travers du réacteur. Cette circulation, ou ce balayage du réacteur avec ledit fluide de balayage, permet par exemple d'entraîner des molécules organiques volatilisées du réacteur vers un instrument d'analyse. Ce balayage permet également d'évacuer des solvants évaporés à l'extérieur du réacteur ou encore de nettoyer et/ou de vidanger le réacteur en éliminant des impuretés volatiles susceptibles d'être présentes dans le réacteur.

Le « fluide de balayage » peut être un gaz ou un liquide. Selon l'invention, le fluide de balayage peut être un gaz inerte. Le gaz inerte peut être par exemple de l'hélium, de l'azote, de l'argon, du dioxyde de carbone, etc.

Selon l'invention, le DPE peut comprendre en outre un moyen de chauffage du fluide de balayage. Il peut par exemple s'agir d'une cartouche chauffante permettant de chauffer le fluide en sortie du réservoir ou d'une gaine thermostatée entourant la canalisation connectant ledit réservoir à l'entrée étanche du DPE. On peut par exemple citer la cartouche chauffante commercialisée par la société RS Components, France, sous la référence 731-243.

Selon l'invention, le DPE peut comprendre en outre un moyen permettant de faire le vide ou d'appliquer une pression dans le réacteur. On entend par moyen permettant d'appliquer une pression dans le réacteur, tout moyen permettant de mettre l'intérieur du réacteur sous une pression de 0,1 à 2.10⁵ kPa, par exemple de 0,1 à 200 kPa, par exemple de 10 à 100 kPa. Parmi les moyens permettant d'appliquer une pression utilisable, on peut citer par exemple une pompe à vide, un générateur de pression, par exemple un compresseur pneumatique, un compresseur électrique, un compresseur à membranes, une sortie connectant le réacteur à un environnement extérieur au réacteur permettant par exemple de mettre le réacteur à la pression dudit environnement extérieur au réacteur ou à une pression intermédiaire, etc. A titre d'exemple, pour les applications spatiales sur Mars, le moyen permettant d'appliquer une pression peut être une sortie commandée par une vanne donnant sur l'environnement de Mars et permettant d'appliquer au réacteur une pression inférieure ou égale à la pression martienne qui est de 0,7 kPa. Parmi les pompes utilisables, on peut citer par exemple une pompe rotative, une pompe à piston, une pompe à palettes, une pompe à bélier, une pompe à godets, une pompe péristaltique, une pompe à vide, une pompe à engrenages, une pompe Valdès, une pompe par Airlift, une pompe hydraulique, une pompe pneumatique, ou toute autre pompe connue de l'homme du métier.

Par exemple, le moyen permettant de faire le vide ou d'appliquer une pression dans le réacteur, appelé également moyen de commande de la pression, peut permettre d'optimiser les conditions de traitement de l'échantillon, notamment les conditions d'extraction, de fonctionnalisation et de volatilisation des molécules organiques de l'échantillon. En particulier, le moyen de commande de la pression présente l'avantage d'améliorer et d'optimiser les rendements d'extraction par thermodésorption grâce à une bonne maîtrise de la pression dans le réacteur.

Selon l'invention, le DPE peut comprendre en outre un moyen d'aspiration de fluides ou de molécules organiques volatilisées. Ce moyen permet d'évacuer par exemple les fluides issus de l'évaporation d'une substance liquide, les fluides de balayage, les fluides de refroidissement ou les molécules organique volatilisées susceptibles d'être présentes dans le réacteur. Cette évacuation permet par exemple de vidanger ou de nettoyer le réacteur. Il peut par exemple s'agir du moyen permettant de faire le vide précité. Parmi les moyens d'aspiration utilisables, on peut citer par exemple une pompe à vide, une sortie connectant le réacteur à un environnement extérieur au réacteur dont la pression est inférieure à celle du réacteur.

Selon l'invention, le DPE peut comprendre en outre un moyen de nettoyage du réacteur permettant d'évacuer le solide, les fluides ou les particules susceptibles d'être présentes dans le réacteur et de nettoyer le réacteur. Par exemple, le nettoyage du réacteur peut être réalisé par pyrolyse et évacuation des impuretés volatilisées issues de la pyrolyse par un balayage par un fluide de balayage. Ou encore, le nettoyage du réacteur peut être réalisé par évacuation du solide, par exemple par le moyen d'introduction du DPE ou par un sas de vidange, puis lavage, manuel ou automatisé, avec un ou plusieurs solvant(s), comme par exemple de l'éthanol ou de l'acétone, éventuellement sous ultrasons, et éventuellement suivi d'un séchage par un gaz inerte. Avantageusement, ledit moyen de nettoyage permet le nettoyage du réacteur en moins d'une heure. Le moyen de nettoyage du DPE de l'invention permet de garantir la propreté du réacteur avant ou après chaque utilisation du DPE. Par exemple, il peut permettre une grande propreté du réacteur avec une concentration d'impuretés résiduelles inférieure à 10⁻¹⁴ mole/cm³, de préférence inférieure à 10⁻¹⁵ mole/cm³. Cette propreté peut être évaluée à l'aide d'un balayage du réacteur et d'une analyse par CPG-SM ayant par exemple une limite de détection de 10⁻¹⁴ mole/cm³, par exemple de 10⁻¹⁵ mole/cm³. Ainsi, le moyen de nettoyage du DPE de l'invention permet au DPE d'être réutilisable plusieurs fois.

Avantageusement, le dispositif de préparation d'échantillon de la présente invention peut comprendre en outre au moins un moyen d'agitation. En effet, celui-ci peut permettre d'améliorer les rendements ou d'accélérer les étapes de préparation d'échantillon, notamment au cours des étapes d'extraction et de fonctionnalisation.

On entend par « moyen d'agitation » au sens de la présente invention tout moyen approprié pour permettre l'agitation du contenu du réacteur comprenant l'échantillon. Il peut s'agir par exemple d'un agitateur magnétique, d'un agitateur mécanique, d'une sonde à micro-ondes, d'un moyen permettant d'appliquer des ultrasons à l'intérieur du réacteur, comme par exemple une cuve à ultrasons dans laquelle est placé le DPE ou une sonde à ultrasons placée par exemple sur ou dans le réacteur.

Avantageusement, le moyen d'agitation peut être une sonde appliquant des ultrasons à l'intérieur du réacteur. Cette sonde résiste de préférence aux conditions de température et de pression appliquées dans le cadre de l'utilisation du DPE. Ainsi, la sonde à ultrasons peut permettre une agitation thermique d'un mélange solide-liquide ou liquide-liquide contenu dans le réacteur. Par exemple, la sonde à ultrasons peut être disposée dans un orifice du réacteur débouchant à l'intérieur de celui-ci. Il peut par exemple s'agir d'une sonde à ultrasons comprenant un générateur ultrasonique, un transducteur piézoélectrique, un adaptateur et une sonotrode. On peut par exemple citer la sonotrode commercialisée par la société Hielscher USA sous la référence UP50H. En outre, la sonde à ultrasons peut être équipée d'un variateur de fréquence et/ou d'intensité des ultrasons. Ainsi, en faisant varier la fréquence et l'intensité des ultrasons ou en optimisant les valeurs de fréquence et d'intensité des ultrasons, ladite sonde peut permettre d'améliorer le traitement, notamment l'extraction et la fonctionnalisation, de l'échantillon, en termes d'efficacité, de rapidité et/ou de rendement. Ce moyen d'agitation présente également l'avantage d'être peu encombrant et donc plus compatible avec des contraintes d'encombrement du DPE, par exemple pour être transportable facilement ou pour une utilisation extraterrestre.

Avantageusement, le dispositif de préparation d'échantillon de la présente invention peut comprendre en outre au moins un moyen de rétention de l'échantillon solide dans le réacteur, agencé pour retenir l'échantillon lors de la circulation d'un fluide de balayage. Ainsi, le moyen de rétention de l'échantillon solide permet notamment d'éviter les pertes d'échantillon solide au cours du fonctionnement du dispositif, et en particulier lors du balayage du réacteur par un fluide de balayage. Ce moyen de rétention permet également d'éviter que des particules de l'échantillon solide n'atteignent le ou les instruments d'analyse.

Le moyen de rétention peut être par exemple une grille ou un matériau de protection de porosité inférieure à la granulométrie minimale de l'échantillon placée en sortie des fluides de balayage afin d'empêcher le passage du solide. Le moyen de rétention peut être par exemple une couche de fritté, de laine de verre ou de coton disposée à la sortie du réacteur pour éviter, lors du balayage par le fluide de balayage, d'entraîner une partie de l'échantillon solide. On notera, toutefois, que compte tenu des faibles pressions de gaz utilisées lors du balayage, l'utilisation d'un tel moyen de rétention n'est pas obligatoire. En outre, le moyen de rétention peut être nettoyable et/ou remplaçable en cas d'obstruation par le solide.

Avantageusement, le dispositif de préparation d'échantillon de la présente invention peut comprendre en outre au moins un moyen étanche d'introduction d'une substance liquide dans le réacteur.

On entend par « substance liquide » au sens de la présente invention tout liquide ou mélange de liquides utilisable dans le cadre de l'application de DPE. Il peut s'agir par exemple d'un solvant, d'un mélange de solvants ou d'un réactif liquide, comme par exemple un agent de fonctionnalisation, celui-ci étant éventuellement en mélange dans un solvant.

On entend par « moyen étanche d'introduction d'une substance liquide » au sens de la présente invention tout moyen étanche connu de l'homme du métier permettant d'introduire une substance liquide dans un réacteur. Le moyen étanche d'introduction d'une substance liquide dans le réacteur peut être par exemple un septum constitué d'une matière à la fois perforable et auto-obturable. Ainsi, le septum peut être traversé par une aiguille ou une seringue permettant l'injection de ladite substance liquide. Le septum peut être constitué de toute matière perforable et auto-obturable connue de l'homme du métier, par exemple du silicone, du polyuréthane, du caoutchouc, etc. Le moyen étanche d'introduction d'une substance liquide dans le réacteur peut également être un conduit muni d'une vanne contrôlant l'introduction de ladite substance liquide dans le réacteur. La vanne peut éventuellement être connectée à une unité de commande qui permet de commander et de contrôler l'injection de substance liquide et le volume de substance liquide à injecter.

Selon un autre mode de réalisation, le moyen d'introduction d'une substance liquide peut être composé d'un compartiment, formé par exemple dans le fond du réacteur, contenant la substance liquide et séparé de l'échantillon de solide par un film de matériau perforable. Ainsi, ladite subtance liquide peut être mise en contact avec l'échantillon à l'aide d'un système de perforation du film. Dans un autre mode de réalisation, le moyen d'introduction d'une substance liquide peut être composé d'une capsule contenant une substance liquide, en matériau thermodestructible et disposée dans le réacteur. Ainsi, ladite substance liquide peut être mise en contact avec l'échantillon par chauffage.

Dans un mode de réalisation particulier de l'invention, le dispositif de préparation d'échantillon peut comprendre en outre au moins un réservoir destiné à contenir une substance liquide, connecté audit moyen étanche d'introduction d'une substance liquide. Celui-ci permet d'approvisionner directement le réacteur en substance liquide et peut être par exemple utile pour des applications spatiales.

Selon l'invention, le moyen de contrôle de la température peut être un capteur de température positionné dans le réacteur. Selon l'invention, le moyen de commande et de contrôle de la température peut être un thermocouple associé avec le moyen de chauffage du réacteur.

Selon l'invention, le moyen de contrôle de la pression peut comprendre un capteur de pression positionné dans le réacteur, par exemple connecté à un orifice formé dans le réacteur ou déporté légèrement en aval de celui-ci, par exemple sur un conduit allant du réacteur vers un instrument d'analyse. Ainsi, la pression peut être mesurée soit directement dans le réacteur, soit à la sortie du réacteur, par exemple jusqu'à 3 cm en aval du réacteur, par exemple à 2 cm en aval du réacteur. Selon l'invention, le capteur de pression résiste de préférence aux conditions de température et de pression appliquées dans le cadre de l'utilisation du DPE. On peut par exemple citer le capteur de pression à fibre optique commercialisé par la société FISO Technologies (Canada) sous la référence FOP-MH, qui supporte la température de 450°C.

Avantageusement, le DPE de l'invention peut comprendre en outre un système d'acquisition des valeurs de température et de pression, ledit système permettant de contrôler, de suivre et d'enregistrer en continu la température et la pression dans le réacteur au cours de l'utilisation du DPE. Ce système permet ainsi d'acquérir et de traiter les données de température et de pression sur un ordinateur, et/ou, lorsqu'il est couplé à un moyen de commande de la température et/ou de la pression, de fixer ou réajuster la température et la pression à l'intérieur du DPE. Ce système permet donc de savoir ce qui se passe dans le DPE à un instant donné et d'optimiser toutes les conditions. Parmi les systèmes utilisables, on peut citer par exemple un Equipement de Test et de Contrôle (ETC), permettant l'acquisition de la température et de la pression, ainsi que leur contrôle. Cet équipement de Test et de Contrôle intègre notamment le Logiciel LabVIEW (Signal Express avec SSP), une carte d'acquisition (NI PCI-4351V) de températures et de pression et une alimentation stabilisée avec une entrée pour le pilotage analogique. Cet équipement intègre également une phase de programmation de l'interface Homme / Machine et de la gestion de la régulation thermique permettant le stockage des données.

La forme du réacteur peut être quelconque. Le réacteur peut par exemple être sous forme de disque, sous forme cylindrique, parallélipipédique, pyramidal, conique, tronconique, etc. Ce dispositif peut être en une seule partie ou en plusieurs parties, par exemple en deux parties, par exemple sous forme d'un contenant et de son couvercle.

Selon l'invention, le réacteur est de préférence constitué d'un matériau conducteur de chaleur de façon à permettre des montées et descentes rapides de la température à l'intérieur du réacteur. Il peut s'agir de tout matériau conducteur permettant de résister aux conditions de température et de pression appliquées dans le cadre de l'utilisation du DPE. Des intervalles de température et de pression applicables dans le cadre de l'utilisation du DPE sont par exemple décrits ci-dessous. Avantageusement, le réacteur peut être constitué d'un ou plusieurs matériaux choisis dans le groupe comprenant l'inox, le cuivre, le titane, l'acier ou la céramique.

Le volume du réacteur peut être choisi suivant la quantité d'échantillon à préparer en vue de l'analyse des molécules organiques qu'il contient. Par exemple, pour un volume d'échantillon de 0,1 à 4 cm³, le volume de l'enceinte du réacteur peut être de 2 à 10 mL.

Selon l'invention, la forme du fond du réacteur peut être choisie de façon à permettre l'optimisation de la préparation de l'échantillon, par exemple en permettant le placement de l'échantillon dans un lieu précis de l'intérieur du réacteur ou en maximisant les surfaces de contact entre l'échantillon et une substance liquide introduite dans le réacteur. De préférence, la forme du fond du réacteur permet de concentrer l'échantillon au fond du réacteur et de favoriser l'immersion totale de l'échantillon dans la substance liquide. Par exemple, le fond du réacteur peut être sous forme plane, courbe, sous forme de cupule, sous forme conique, etc. En outre, la surface de l'intérieur du réacteur est de préférence lisse afin d'éviter que des poches de fluide ou de gaz subsistent après le balayage par un fluide de balayage.

Selon l'invention, les parois internes du réacteur sont avantageusement en matériau inattaquable, en particulier lorsque les conditions d'utilisation du réacteur peuvent être corrosives. Il peut s'agir par exemple d'un métal inattaquable ou recouvert d'un revêtement inattaquable afin d'éviter que les substances liquides introduites dans le réacteur n'attaquent les parois du réacteur. Parmi les revêtements utilisables, on peut citer par exemple le traitement Silcosteel (marque déposée) développé par la société Restek (USA) et décrit dans le brevet US N° 6,511,760 (ref 5). Ce revêtement a une épaisseur de 300Å à 350Å et supporte une température maximale de 600°C.

Les moyens de chauffage et de refroidissement très rapides du dispositif de la présente invention permettent de maîtriser parfaitement les temps durant lesquels le réacteur est soumis aux hautes ou basses températures, puisque les paliers intermédiaires de montée ou de descente de température sont maîtrisés et atteints très rapidement. Ainsi, le dispositif de la présente invention présente l'avantage de permettre l'extraction de molécules organiques à des températures extrêmes (par exemple à des températures supérieures à 250°C, par exemple supérieures à 350°C, par exemple supérieures à 450°C) pendant des durées bien maîtrisées, tout en évitant leur dégradation, notamment par refroidissement rapide après l'extraction.

Selon l'invention, le moyen de chauffage dudit réacteur peut être choisi dans le groupe comprenant une cartouche chauffante, une plaque chauffante, une résistance électrique, un balayage de gaz chaud, un chauffage radiatif, par exemple par induction ou par halogène. Avantageusement, le moyen de chauffage peut être une cartouche chauffante. La cartouche chauffante peut être composée d'une résistance électrique, par exemple de forme cylindrique, éventuellement protégée par un blindage, par exemple en acier inoxydable. Afin de permettre une montée rapide de la température du réacteur, la puissance de la cartouche chauffante peut être par exemple supérieure à 400 W (Watt). On peut par exemple citer la cartouche chauffante commercialisée par la société RS Components (France) sous la référence 731-243.

Selon l'invention, le moyen de refroidissement dudit réacteur est une plaque de refroidissement traversée d'un circuit de refroidissement.

On entend par gel ou par fluide « de refroidissement » au sens de la présente invention un gel ou un fluide, par exemple un liquide ou un gaz, permettant le refroidissement rapide du réacteur à une vitesse moyenne de 10 à 50 °C/seconde, par exemple de 25 à 50 "C/seconde. Parmi les liquides ou gels de refroidissement, on peut citer par exemple l'eau, l'azote liquide, le CO₂ ou les mélanges réfrigérants comprenant par exemple de la glace ou de la carboglace. Parmi les gaz de refroidissement, on peut citer par exemple l'air froid, le CO₂, l'azote, l'argon, l'hélium, etc.

On entend par « circuit de refroidissement » au sens de la présente invention un circuit comprenant un ou plusieurs conduits dans lesquels sont mis en circulation des fluides de refroidissement, par exemple des liquides ou gaz de refroidissement. Le circuit de refroidissement peut par exemple être compris dans les parois du réacteur ou dans la plaque de refroidissement.

On entend par « plaque de refroidissement » au sens de la présente invention une plaque capable d'absorber la chaleur du DPE par transfert thermique. Il peut s'agir d'une plaque pleine en matériau conducteur de chaleur ou d'une plaque traversée par un circuit de refroidissement.

Avantageusement, le moyen de refroidissement dudit réacteur est une plaque de refroidissement absorbant la chaleur mise en contact direct avec le DPE. La rapidité du refroidissement du réacteur peut dépendre de divers paramètres comme par exemple le matériau conducteur de la plaque de refroidissement et/ou du DPE, l'épaisseur de la plaque de refroidissement, la surface de contact la plaque de refroidissement avec le DPE, la qualité du contact de la plaque de refroidissement avec le DPE, le volume du DPE à refroidir, la variation de température à effectuer. Les calculs de transfert de chaleur peuvent être effectués en utilisant par exemple un coefficient d'échange H de 10 000 W/m²/°C. Parmi les matériaux conducteurs utilisables, on peut citer par exemple le cuivre, l'inox ou le titane. En outre, pour réaliser un refroidissement rapide à une vitesse moyenne de 10 à 50 °C/seconde, les dimensions de la plaque de refroidissement peuvent être avantageusement, pour un volume de réacteur de 2 à 10 mL, de 20x20x10 (mm) à 80x80x40 (mm), de préférence de 35x35x15 (mm) à 45x45x25 (mm). Avantageusement, le dispositif peut être maintenu sur la plaque de refroidissement avec une force de serrage de 500 à 2000 N de préférence de 900 à 1600 N afin de réaliser un bon contact et d'optimiser le refroidissement. Le mode de serrage peut par exemple être réalisé au moyen de pinces de serrage, ou d'un système de mise en pression comme par exemple une presse. Ce moyen de refroidissement présente notamment l'avantage de permettre une descente en température jusqu'à une température de - 50°C et même au-delà grâce à un balayage de la plaque froide par un fluide froid tel que _de l'azote liquide ou de CO₂. Un exemple de plaque froide est donné dans la partie Exemples.

Selon un deuxième aspect, l'invention concerne également un système d'analyse comprenant au moins un dispositif de préparation d'échantillon (DPE) selon l'invention et au moins un instrument d'analyse connecté en aval dudit DPE. Ce système d'analyse au sens de la présente invention permet la préparation de l'échantillon mais également l'analyse chimique des molécules organiques qu'il contient.

On entend par « instrument d'analyse » au sens de la présente invention tout appareil permettant l'analyse chimique de molécules organiques, par exemple l'analyse moléculaire, chirale, et même isotopique (cette dernière pouvant être obtenue au moyen d'un spectromètre de masse par exemple). L'instrument d'analyse peut par exemple être choisi dans le groupe comprenant un spectromètre infra-rouge, un chromatographe, utilisant par exemple la chromatographie en phase gazeuse (CPG) ou la chromatographie liquide haute performance (CLHP), un spectromètre de masse (SM). L'instrument d'analyse peut éventuellement être un instrument miniaturisé par exemple pour des applications spatiales du système d'analyse. On peut par exemple citer l'instrument d'analyse par chromatographie en phase gazeuse décrit dans le document de Sternberg et al., Encyclopaedia of Separation Science, Academic Press, London, (2006) (ref 6).

Selon l'invention, le système d'analyse peut comprendre en outre un détecteur connecté en aval dudit instrument d'analyse. Parmi les détecteurs utilisables on peut citer par exemple un spectrophotomètre, un spectromètre infra-rouge (IR), un spectromètre de masse (SM), un détecteur à ionisation de flamme (FID ou « Flame ionization detector »), un détecteur à conductivité thermique (TCD ou « Thermal Conductivity Detector »).

Avantageusement, l'instrument d'analyse peut être un chromatographe en phase gazeuse. De préférence, l'instrument d'analyse peut être un chromatographe en phase gazeuse (CPG) couplé à un spectromètre de masse (SM) ou à un détecteur, qui peut être un détecteur classique de CPG. On peut citer par exemple l'instrument, d'analyse décrit dans le document de Sternberg et al., Encyclopaedia of Separation Science, Academic Press, London, (2006) **(ref 6).**

Selon l'invention, le système d'analyse peut comprendre plusieurs DPE connectés ou connectables à l'instrument d'analyse. Les DPE peuvent par exemple être connectés et déconnectés un par un à l'instrument d'analyse, afin de réaliser successivement, pour chaque DPE, le traitement et l'analyse de l'échantillon qu'il contient. Les DPE peuvent être connectés et déconnectés manuellement ou automatiquement à l'instrument d'analyse à l'aide de tout moyen de connexion approprié, par exemple au moyen de canalisations munies de vannes.

Le système d'analyse peut comprendre en outre, en aval du DPE et en amont de l'instrument d'analyse, un moyen d'injection des molécules organiques dans l'instrument d'analyse. Il peut s'agir par exemple d'un thermodésorbeur ou d'une boucle d'échantillonnage permettant l'injection des molécules organiques par l'intermédiaire d'un gaz inerte. Cette boucle suivant son volume permet d'injecter ponctuellement des volumes de gaz et de molécules organiques allant de 10 à 2000 µL. Une telle boucle est par exemple décrite dans le document Meunier et al., Advances in Space Research (2007) 39, 3, pp 337-344 (ref 7). Parmi les injecteurs thermodésorbeurs utilisables, on peut citer par exemple l'injecteur OPTIC 3 commercialisé par la société ATAS GL International BV (Pays-Bas).

Le système d'analyse peut comprendre, en outre, un circuit de balayage des molécules organiques comprenant :
- un réservoir de fluide de balayage, comme par exemple une bouteille de gaz inerte ou un réacteur contenant le fluide de balayage ;
- une canalisation connectant ledit réservoir de fluide de balayage à l'entrée étanche du DPE ; et
- une canalisation connectant la sortie du DPE à l'instrument d'analyse.

Le système d'analyse peut être un système amovible, c'est-à-dire qui peut être monté, démonté et/ou déplacé ou encore dont les éléments constituant le système d'analyse peuvent être séparés. En outre, il présente l'avantage d'être facilement transportable sur un lieu de prélèvement d'échantillon.

En résumé, le DPE de la présente invention présente l'avantage de permettre en un seul lieu, le réacteur, et avec un minimum d'étapes la préparation d'un échantillon et notamment l'extraction, la fonctionnalisation et la volatilisation de molécules organiques présentes dans un échantillon, ainsi que leur transfert vers un instrument d'analyse. Il facilite ainsi grandement les procédures de préparation d'échantillon en vue de l'analyse des molécules qu'il contient. En outre, en effectuant toutes ces étapes en un même lieu, il permet d'éviter les pertes liées aux transferts de l'échantillon dans différents réacteurs et d'obtenir de bons rendements.

En outre, le DPE de la présente invention permet un parfait contrôle de la propreté et de la fiabilité de l'analyse de l'échantillon. En effet, une fois l'échantillon introduit, il n'y a plus de risque de pollution jusqu'à l'analyse. Il permet ainsi une meilleure fiabilité des résultats d'analyse.

Le DPE de la présente invention permet également l'analyse de traces dans différentes matrices et cela avec de faibles masses d'échantillons.

En outre, il peut permettre l'analyse des molécules organiques contenues dans un échantillon sans dénaturation de leurs propriétés chimiques et physiques, comme par exemple l'asymétrie ou la chiralité des molécules organiques.

Le DPE de l'invention présente également l'avantage d'être multifonctionnel, et permet de réaliser diverses méthodes et procédés, comprenant par exemple une ou plusieurs étapes choisies dans le groupe comprenant l'extraction, la fonctionnalisation, la volatilisation et le transfert vers un instrument d'analyse de molécules organiques présentes dans un échantillon. En outre, il peut également combiner la fonctionnalité de pyrolyseur, par exemple pour le nettoyage du réacteur ou, si nécessaire, pour réaliser des analyses complémentaires des molécules organiques ainsi dégradées. En effet, il permet de réaliser des méthodes de pyrolyse et de thermochimiolyse, par exemple jusqu'à des températures de 1000°C, par exemple jusqu'à des températures de 600 °C. Ces fonctionnalités sont en effet permises grâce au moyen de chauffage du réacteur à une vitesse moyenne de 10 à 50°C/seconde que le DPE comprend. On peut noter que le dispositif de la présente invention présente l'avantage de ne pas se limiter à la mise en oeuvre d'une simple pyrolyse de molécules organiques, contrairement à la plupart des dispositifs de l'art antérieur.

Avantageusement, le DPE de l'invention permet d'appliquer à l'échantillon des conditions de température et de pression extrêmes. Le DPE de l'invention peut être utilisé par exemple pour des intervalles de température allant de -100°C à 1000°C, par exemple de -50°C à 600°C, par exemple de 0°C à 600°C. Par ailleurs, le DPE de l'invention peut être utilisé pour des intervalles de pression allant de 0,1 à 2.10⁵ kPa, par exemple de 0,1 à 200 kPa.

Enfin, le DPE de la présente invention peut être compatible avec n'importe quelles techniques d'analyse en phase gazeuse et adaptable avec des techniques séparatives comme par exemple la HPLC, l'électrophorèse capillaire, etc.

Le dispositif de préparation d'échantillon permet par exemple la mise en oeuvre des procédés (I) ou (II) suivants d'extraction, de fonctionnalisation et de volatilisation de molécules organiques :

Le procédé (I) dit « un lieu - une étape » (ou « one pot - one step ») peut comprendre les étapes suivantes :
(Ia) introduire dans un réacteur un échantillon solide prélevé
(Ib) mettre le réacteur sous une pression de 0,1 à 200 kPa ;
(Ic) élever en moins de 30 secondes, de préférence en moins de 15 secondes, la température du réacteur de la température de l'environnement du réacteur à une température T_{d} allant de 250°C à 600°C ;
(Id) maintenir le réacteur à la température T_{d} pendant une durée D_{d} inférieure à 15 minutes afin de désorber par thermo-désorption les molécules organiques contenues dans l'échantillon solide ;
(Ie) diminuer, en moins de 30 secondes, de préférence en moins de 15 secondes, la température du réacteur de la température T_{d} à la température T_{f} allant de 50°C à 100°C ;
(If) ajouter dans le réacteur au moins un agent de fonctionnalisation et au moins un solvant organique, et maintenir le réacteur à la température T_{f} pendant une durée D_{f} inférieure à 1 heure, afin de faire réagir le mélange et de fonctionnaliser les molécules organiques désorbées ; et
(Ig) chauffer le réacteur à une température Tᵥ allant de 140°C à 300°C pendant une durée Dᵥ inférieure à 1 heure afin de volatiliser les molécules organiques fonctionnalisées, la température Tᵥ étant de préférence atteinte en moins de 30 secondes, par exemple en moins de 15 secondes.

Le procédé (II) dit « one pot - two steps » peut comprendre les étapes suivantes :
(IIa) introduire dans un réacteur un échantillon solide prélevé
(IIb) introduire dans ledit réacteur un solvant organique ;
(IIc) mettre le mélange sous agitation à une température Tₑ allant de 20°C à 100°C pendant une durée De inférieure à 1 heure afin d'extraire des molécules organiques du solide ;
(IId) éliminer le solvant, par exemple le solvant peut être évaporé et évacué à l'aide d'un balayage du réacteur par un gaz inerte, le gaz inerte étant de préférence à une température de 30°C +/- 5°C;
(IIf) ajouter dans le réacteur au moins un agent de fonctionnalisation et au moins un solvant organique, et maintenir le réacteur à la température T_{f} allant de 50°C à 100°C pendant une durée D_{f} inférieure à 1 heure afin de fonctionnaliser les molécules organiques extraites ;
(IIg) chauffer le réacteur à une température Tᵥ allant de 140°C à 300°C pendant une durée Dᵥ inférieure à 1 heure afin de volatiliser les molécules organiques fonctionnalisées, la température Tᵥ étant de préférence atteinte en moins de 30 secondes, par exemple en moins de 15 secondes.

Chacun des procédés (I) ou (II) peut comprendre en outre, pendant ou après l'étape (g) de volatilisation, une étape de transfert des molécules volatilisées vers un instrument d'analyse.

Les étapes d'extraction, de fonctionnalisation, de volatilisation et de transfert réalisables à l'aide du DPE de l'invention sont décrites ci-dessous.

### Extraction de molécules organiques

On entend par « extraction » la séparation des molécules organiques de la matrice solide qui les contient. Par exemple, dans le cas d'un échantillon solide, les molécules organiques absorbées ou adsorbées à sa surface peuvent être fortement liées au solide et peuvent être très difficiles à extraire, d'où la nécessité de recourir à des méthodes d'extraction efficaces.

Le DPE de la présente invention permet de réaliser l'extraction de molécules organiques dans le réacteur. Par exemple, il peut s'agir d'une extraction à l'aide d'un solvant ou d'une extraction par thermo-désorption.

L'extraction à l'aide d'un solvant peut être effectuée en agitant l'échantillon dans un solvant à la température d'extraction Tₑ favorisant la solubilisation des molécules organiques dans le solvant et pendant une durée De. Des moyens d'agitation utilisables avec le DPE sont décris ci-dessus.

Le solvant peut être tout solvant connu de l'homme du métier permettant la solubilisation des molécules organiques présentes dans l'échantillon solide. Par exemple, le solvant peut être choisi dans le groupe comprénant l'eau, les alcanes en C₁ à C₈, linéaires, ramifiés ou cycliques, les alcools R^{S}-OH où R^{S} est un radical alkyle en C₁ à C₈, par exemple l'isopropanol, les cétones R^{S1}-C(=O)-R^{S2}, les éthers R^{S1}-O-R^{S2}, où R^{S1} et R^{S2} sont indépendamment des radicaux alkyles en C₁ à C₈, formant éventuellement un cycle, le tétrahydrofurane, le dioxane, l'acétonitrile, le diméthylformamide (DMF), le diéthylformamide, le diméthylsulfoxyde, le toluène, l'acide acétique, l'acide formique, le dichlorométhane, le chloroforme, le dichloroéthane, l'acétate d'éthyle, ou un mélange de ceux-ci. On entend par « alkyle » un radical carboné linéaire, ramifié ou cyclique, saturé ou insaturé, éventuellement substitué, comprenant 1 à 8 atomes de carbone, par exemple 1 à 6 atomes de carbone. On peut noter à titre d'exemple que le DMF et l'eau permettent la solubilisation et l'extraction de molécules organiques comme les acides aminés tandis que l'isopropanol permet la solubilisation et l'extraction d'autres molécules organiques cibles. Le volume de solvant utilisé peut être choisi de façon à immerger totalement l'échantillon.

La température d'extraction Tₑ peut être de 20°C à 100°C, par exemple de 50°C à 80°C.

La durée De peut être inférieure à 1 heure, par exemple de 10 à 30 minutes, par exemple de 10 à 20 minutes.

La pression Pₑ peut être de 0,1 à 2.10⁵ kPa. Par exemple, la pression Pₑ aller de 0,1 à 200 kPa, par exemple, de 10 à 200 kPa. Des moyens d'application d'une pression utilisable avec le DPE sont décris ci-dessus.

L'extraction à l'aide d'un solvant peut éventuellement être réalisée sous agitation par ultrasons ou sous micro-ondes afin de faciliter l'extraction des molécules organiques. Des moyens permettant d'appliquer des ultrasons et micro-ondes avec le DPE sont décris ci-dessus.

Une fois les molécules organiques extraites, le solvant peut être éliminé, par évaporation et/ou par entraînement au moyen d'un gaz inerte, sous l'une ou plusieurs des conditions suivantés :
- à une pression Pₑᵥ allant de 0,1 à 2.10⁵ kPa, par exemple de 0,1 à 200 kPa, par exemple de 10 à 100 kPa.
- à une température Tₑᵥ allant de 25 à 35°C, de préférence de 30°C +/-1°C, afin d'éviter l'évaporation des molécules organiques volatiles qui peuvent également être présentes dans l'échantillon ;
- sous un balayage de gaz inerte à un débit inférieur à 20 mL/minute, par exemple de 1 mL/minute.
les conditions d'évaporation ci-dessus étant réalisées pendant une durée Dₑᵥ inférieure à 40 minutes, par exemple de 15 à 30 minutes. Des moyens de balayage utilisables avec le DPE sont décris ci-dessus.

On entend par « thermo-désorption » une méthode permettant de désorber des molécules organiques absorbées ou adsorbées sur un support solide, par chauffage à une température de désorption T_{d} et à une pression P_{d} pendant une durée D_{d}.

Selon l'invention, le DPE permet d'appliquer une pression P_{d} dans le réacteur allant de 0,1 à 2.10⁵ kPa. La pression P_{d} peut être choisie de façon à faciliter la désorption, par exemple, l'extraction par thermo-désorption peut être effectuée sous pression réduite. Par exemple, la pression P_{d}, peut aller de 0,1 à 100 kPa, par exemple de 10 à 100 kPa. Des moyens d'application d'une pression utilisable avec le DPE sont décris ci-dessus.

Selon l'invention, le DPE permet d'appliquer une température de désorption T_{d} élevée, c'est-à-dire qui peut aller de 250°C à 600°C. Par exemple, la température T_{d} peut être de 300 à 550 °C, par exemple de 300 à 500 °C. Avantageusement, la température T_{d} peut être de 500°C +/-10°C, par exemple de 500°C +/- 5°C.

La température de désorption T_{d} peut être maintenue pendant une durée D_{d} inférieure à 15 minutes, par exemple, inférieure à 5 minutes. Selon un autre mode de réalisation de l'invention, la durée D_{d} peut être de 5 minutes +/- 1 minute.

Le DPE permet éventuellement de réaliser l'extraction par thermo-désorption sous ultrasons ou sous micro-ondes afin de faciliter la désorption des molécules organiques.

Le DPE selon l'invention permet une élévation rapide de température, c'est-à-dire qu'elle peut s'effectuer en moins de 30 secondes, par exemple en moins de 15 secondes. Avantageusement, le DPE de la présente invention permet d'élever la température du réacteur en 15 secondes +/- 10 secondes. Ceci permet de contrôler avec précision le temps pendant lequel l'échantillon est maintenu à température élevée. En effet, au-delà d'un certain temps à température élevée, les molécules peuvent être dégradées. Ainsi, une élévation de température trop lente peut engendrer la dégradation des molécules organiques. De la même façon, le DPE permet une diminution de température rapide, c'est-à-dire qu'elle peut s'effectuer en moins de 15 secondes. Avantageusement, le DPE de la présente invention permet de diminuer la température du réacteur en 15 secondes +/- 10 secondes. Comme décrit précédemment, ceci permet de limiter les risques de dégradation des molécules organiques.

Ainsi, le DPE de l'invention présente l'avantage de pouvoir utiliser la méthode par thermodésorption pour l'extraction de molécules organiques.

Le DPE de l'invention permet une extraction par thermo-désorption rapide et ne nécessitant que quelques minutes. En effet, le DPE de l'invention permet de réaliser une extraction en moins de 20 minutes, par exemple en moins de 15 minutes, par exemple en moins de 10 minutes.

De plus, le DPE permet d'extraire et en particulier de désorber les molécules organiques de la matrice solide qui les contient sans les casser compte tenu de l'application possible de temps de chauffage très courts et de vitesses élevées de variations de températures, même pour des variations importantes de températures.

Le DPE de l'invention permet également d'éviter l'utilisation de solvants, contrairement aux méthodes d'extraction généralement utilisées. En effet, les solvants sont généralement dangereux, néfastes pour l'environnement et/ou coûteux. De plus, ceci permet d'éviter l'étape supplémentaire d'élimination du solvant d'extraction. En outre, ceci permet d'éviter les pertes en molécules organiques extraites qui peuvent parfois être en partie entraînées avec le solvant d'extraction lors de son élimination.

Ainsi, avantageusement, le DPE permet une extraction par thermo-désorption en une seule étape et en consommant moins d'énergie. En outre, il permet d'extraire de faibles quantités de molécules organiques. En effet, les dispositifs et méthodes d'extraction existants sont généralement utilisés pour des quantités relativement importantes d'échantillons et ne sont pas adaptés ni optimisés pour des échantillons présentant des molécules organiques à extraire en faibles quantités ou à l'état de traces.

Le DPE permet une extraction par thermo-désorption en détachant les molécules organiques de la matrice solide de l'échantillon, que ces molécules soient volatiles ou non volatiles. Ainsi, il permet en outre de séparer les molécules organiques volatiles des molécules organiques non-volatiles. En effet, les molécules organiques volatiles se retrouvant à l'état gazeux suite à la thermo-désorption, peuvent être évacuées hors du réacteur par un fluide de balayage, par exemple entraînées vers un instrument d'analyse, tandis que les molécules organiques non-volatiles restent au fond du réacteur pour y subir les étapes suivantes de fonctionnalisation et de volatilisation.

### Fonctionnalisation de molécules organiques extraites :

On entend par « fonctionnalisation » la transformation chimique de molécules organiques en introduisant au moins un groupe fonctionnel afin de modifier leurs propriétés physico-chimiques comme par exemple leur volatilité.

Ainsi, une fois les molécules organiques extraites de l'échantillon solide, elles peuvent être fonctionnalisées afin de les rendre plus volatiles.

La fonctionnalisation des molécules organiques extraites peut être réalisée en ajoutant à l'échantillon au moins un agent de fonctionnalisation et au moins un solvant, et en maintenant le mélange à une température T_{f} pendant une durée D_{f}, afin de faire réagir les molécules organiques extraites avec au moins un agent de fonctionnalisation pour obtenir des molécules organiques fonctionnalisées.

La température T_{f} peut être de 50°C à 100°C. Par exemple, la température T_{f} peut aller de 60 à 90°C, par exemple de 75°C +/- 10°C, par exemple de 75°C +/- 5°C, par exemple de 75°C +/- 1°C.

L'agitation à la température T_{f} peut être maintenue pendant une durée D_{f} inférieure à 1 heure, par exemple pendant une durée D_{f} de 5 à 40 minutes, par exemple de 10 à 30 minutes.

On entend par « agent de fonctionnalisation » un réactif pouvant se substituer à des groupements ou hydrogènes labiles des molécules organiques pour donner des molécules fonctionnalisées. Ces molécules fonctionnalisées sont généralement plus volatiles. En effet, la fonctionnalisation peut diminuer la polarité des molécules organiques et rendre moins fortes les liaisons hydrogène présentes au sein des molécules permettant ainsi leur volatilisation à de plus faibles températures, par exemple de l'ordre de 200°C.

Dans le cadre de la présente invention, parmi les agents de fonctionnalisation utilisables, on peut distinguer les agents de fonctionnalisation permettant ou non la mesure chromatographique de la chiralité des molécules organiques fonctionnalisées. Cette mesure consiste à détecter et à déterminer qualitativement et quantitativement la présence éventuelle de molécules énantiomères dans l'échantillon. En effét, certains agents de fonctionnalisation peuvent par exemple comprendre un centre chirale, ce qui permet après réaction avec une molécule organique de détecter d'éventuels isomères de la molécule organique fonctionnalisée et donc d'identifier si la molécule organique initiale est chirale ou non.

L'agent de fonctionnalisation peut par exemple être choisi dans le groupe comprenant le *N*-Methyl-*N*-[tert-butyldimethyl-silyl]trifluoroacétamide (MTBSTFA), le *N,N-*diméthylformamide diméthylacétal (DMF-DMA), l'hydroxyde de tétraméthylammonium (TMAH), l'anhydride trifluoroacétique (TFAA), l'annhydride heptafluorobutyrique (HFBA), le 2,2,2-trifluoroéthanol, (TFE), le 2,2,3,3,4,4,4-heptafluoro-1-butanol (HFB), le chloroformate de méthyle (MCF), le chloroformate d'ethyle (ECF) ou un mélange de ceux-ci.

Le tableau 1 suivant présente, pour différents agents de fonctionnalisation, les actions ou substitutions possibles avec les groupements des molécules organiques à fonctionnaliser, ainsi que les propriétés ou non de mesurer la chiralité des molécules organiques.

**Tableau 1**

| **Agent de dérivatization** | **action et Propriétés** |
|---|---|
| MTBSTFA | Tous H labile - pas de chiralité |
| TMAH | Tous composés organiques - pas de chiralité |
| DMF-DMA | Acide aminé - chiralité |
| MCF | H labile - chiralité |
| ECF | H labile - chiralité |
| TFAA + TFE | H labile - chiralité |
| HFBA + HFB | H labile - chiralité |

Le solvant peut être tout solvant connu de l'homme du métier permettant la réaction de fonctionnalisation des molécules organiques par l'agent de fonctionnalisation. Par exemple, le solvant peut être choisi dans le groupe comprenant l'eau, les alcanes en C₁ à C₈, linéaires, ramifiés ou cycliques, les alcools R^{S}-OH où R^{S} est un radical alkyle en C₁ à C₆, par exemple l'isopropanol, les cétones R^{S1}-C(=O)-R^{S2}, les éthers R^{S1}-O-R^{S2}, où R^{S1} et R^{S2} sont indépendamment des radicaux alkyles en C₁ à C₆, formant éventuellement un cycle, le tétrahydrofurane (THF), le dioxane, l'acétonitrile, le diméthylformamide (DMF), le diéthylformamide, le diméthylsulfoxyde, le toluène, l'acide acétique, l'acide formique, le dichlorométhane, le chloroforme, le dichloroéthane, l'acétate d'éthyle, ou un mélange de ceux-ci. Le radical « alkyle » est défini précédemment.

Par ailleurs, on peut noter que le MTBSTFA est un agent de fonctionnalisation agressif pouvant avoir des conséquences sur la conception de l'enceinte et que le DMF est un solvant moins agressif que le MTBSTFA. Des matériaux inattaquables utilisables pour les parois internes du réacteur du DPE sont décris ci-dessus.

L'étape de fonctionnalisation peut être réalisée à une pression P_{f} allant de 0,1 à 2.10⁵ kPa. Par exemple, la pression P_{f} peut être de 0,1 à 200 kPa, par exemple de 0,1 à 100 kPa, par exemple de 10 à 100 kPa. Des moyens permettant d'appliquer une pression utilisables avec le DPE sont décris ci-dessus.

La fonctionnalisation des molécules organiques peut éventuellement être réalisée sous agitation. Par exemple, la fonctionnalisation peut être réalisée sous ultrasons ou sous micro-ondes afin de favoriser la réaction de fonctionnalisation des molécules organiques.

La fonctionnalisation nécessite une étape permettant d'atteindre la température de fonctionnalisation T_{f} qui peut être suivie d'une étape de stabilisation de la température où la température T_{f} est maintenue pendant une durée inférieure à 10 minutes, par exemple une durée de 2 à 5 minutes. Cette étape de stabilisation permet de laisser l'échantillon atteindre la température T_{f} avant l'ajout de l'agent de fonctionnalisation.

### Volatilisation de molécules organiques fonctionnalisées :

On entend par « volatilisation » le passage des molécules organiques à l'état gazeux.

Le DPE de la présente invention permet de réaliser la volatilisation, des molécules organiques fonctionnalisées notamment en vue de leur analyse.

L'étape de volatilisation des molécules organiques fonctionnalisées peut réalisée à l'aide du DPE de l'invention par chauffage des molécules organiques de l'échantillon à une température de volatilisation Tᵥ pendant une durée Dᵥ.

La température Tᵥ peut être de 140°C à 300°C. Par exemple, la température Tᵥ peut être de 180 à 200°C. Avantageusement, la température Tᵥ peut être de 200°C +/- 20°C, par exemple de 200°C +/-10°C.

La température Tᵥ peut être maintenue pendant une durée Dᵥ inférieure à 1 heure. Par exemple, la température Tᵥ peut être maintenue pendant une durée Dᵥ de 10 à 30 minutes.

La volatilisation des molécules organiques fonctionnalisées peut être effectuée à une pression Pᵥ allant de 0,1 à 2.10⁵ kPa. Par exemple, la volatilisation peut être réalisée sous pression réduite, par exemple à une pression Pᵥ allant de 0,1 à 200 kPa, par exemple de 10 à 100 kPa. Des moyens permettant d'appliquer une pression utilisable avec le DPE sont décris ci-dessus.

L'étape de volatilisation peut comprendre en outre une étape de d'élévation de température à la température Tᵥ. L'élévation de température peut être rapide, c'est-à-dire que la température Tᵥ peut être atteinte en moins de 30 secondes, par exemple en moins de 15 secondes. Avantageusement, l'élévation de la température de l'échantillon à la température Tᵥ peut s'effectuer en une durée de 15 secondes +/- 10 secondes.

On peut noter que l'étape de volatilisation peut être suivie en contrôlant la pression dans le réacteur au moyen d'un capteur de pression. En effet, la mesure de la pression peut être un indicateur de la quantité de molécules organiques volatilisées.

Ainsi, quelle que soit la méthode d'extraction utilisée, le DPE de l'invention permet d'effectuer en un même lieu l'extraction (à l'aide d'un solvant ou par thermo-désorption) et la fonctionnalisation des molécules organiques extraites et la volatilisation des molécules fonctionnalisées, directement à partir de l'échantillon solide. Le DPE de l'invention permet également de transférer vers un instrument d'analyse les molécules organiques volatilisées afin de les analyser.

### Transfert de molécules organiques volatilisées

Le transfert des molécules organiques volatilisées peut être effectué après ou pendant l'étape de volatilisation, par exemple au fur et à mesure de l'apparition de molécules organiques volatilisées dans le réacteur.

Le transfert des molécules organiques volatilisées peut être par exemple effectué par aspiration ou par entraînement au moyen d'un gaz inerte. De préférence, le transfert peut être réalisé par entraînement au moyen d'un balayage par un gaz inerte afin de pousser les molécules organiques volatilisées vers le système d'analyse. Des gaz inertes et des moyens de balayage utilisables avec le DPE sont par exemple décris ci-dessus.

Le balayage par le gaz inerte peut être réalisé à la température de volatilisation Tᵥ pendant une durée Dₜ inférieure à 40 minutes, par exemple de 10 à 30 minutes.

Le balayage par le gaz inerte peut être réalisé à une pression Pₜ allant de 0,1 à 2.10⁵ kPa, par exemple de 0,1 à 200 kPa, par exemple de 0,1 à 100 kPa, par exemple de 10 à 100 kPa. Des moyens permettant d'appliquer une pression utilisables avec le DPE sont décris ci-dessus.

Le balayage par le gaz inerte peut être continue. Par exemple, il peut s'agir d'un balayage avec un écoulement laminaire. Par exemple, le balayage peut être effectué à un débit allant de 1 à 10 mL/minute.

D'autres avantages pourront encore apparaître à l'homme du métier à la lecture des exemples non-limitatifs ci-dessous, illustrés par les figures annexées.

### Brève description des figures

- la figure 1 est une vue schématique éclatée d'un dispositif de préparation d'échantillon (DPE) selon l'invention ;
- la figure 2 est une vue schématique latérale du DPE de la figure 1 ;
- la figure 3 est une vue schématique, de dessus, du DPE de la figure 1 ;
- la figure 4 est une vue schématique en coupe transversale du DPE de la figure 1 ;
- la figure 5 est une vue schématique, en perspective, de l'élément inférieur (2) du réacteur du DPE selon l'invention ;
- la figure 6 est une vue schématique, en coupe de l'élément inférieur (2) du réacteur du DPE ;
- la figure 7 est une vue schématique, en perspective, de l'élément supérieur du réacteur du DPE ;
- la figure 8 est une vue schématique, de dessus, de l'élément supérieur du réacteur ;
- la figure 9 est une vue schématique latérale de l'élément supérieur du réacteur ; et
- la figure 10 est une vue schématique d'un système d'analyse d'échantillon selon l'invention.
- La figure 11 représente le DPE couplé à une plaque de refroidissement au moyen d'une presse: La figure 11.a) est une vue schématique latérale de l'ensemble constitué de la presse, du DPE et de la plaque froide ; La figure 11.b) est une vue schématique de dessus de l'ensemble constitué de la presse, du DPE et de la plaque froide ; La figure 11.c) est une vue schématique en perspective de l'ensemble constitué de la presse, du DPE et de la plaque froide.
- La figure 12 représente le chronogramme synthétisant les différents cycles de température en fonction du temps de l'expérience pour l'expérimentation « un lieu / deux étapes ».
- La figure 13 représente le chromatogramme issu de l'analyse chromatographique couplée à la spectrométrie de masse de l'échantillon de 0,5 g de sol du désert d'Atacama après le procédé « un lieu / deux étapes ».
- La figure 14 représente le chronogramme synthétisant les différents cycles de température en fonction du temps de l'expérience pour l'expérimentation « un lieu / une étape »
- La figure 15 représente le chromatogramme issu de l'analyse chromatographique couplée à la spectrométrie de masse de l'échantillon de 0,5 g de sol du désert d'Atacama après le procédé « un lieu / une étape ».

### EXEMPLES

### Exemple 1 : Dispositif de Préparation d'Echantillon

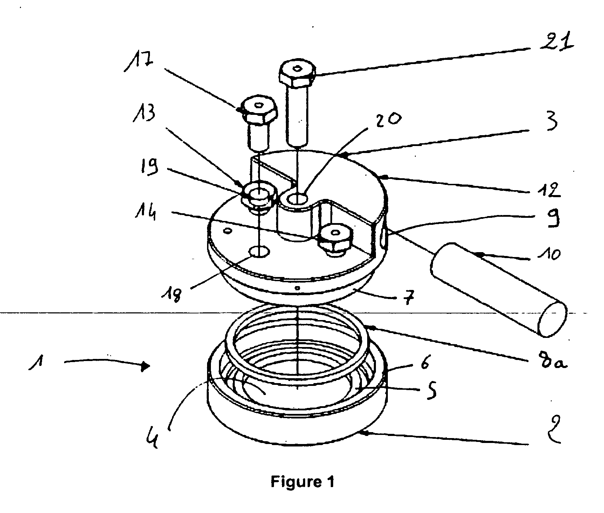

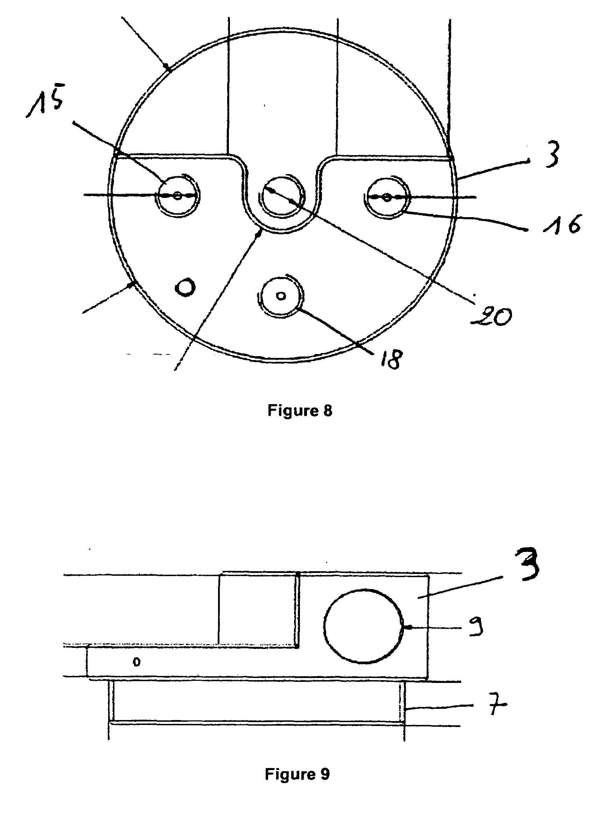

Un exemple de dispositif de préparation d'échantillon selon la présente invention a été réalisé et est illustré par les figures 1 à 11.

La figure 1 représente un dispositif (1) de préparation d'échantillon (DPE) selon l'invention, en vue éclatée. Le DPE comprend un réacteur étanche comportant un élément inférieur (2) (contenant) et un élément supérieur (3) (couvercle) qui peuvent être solidarisés ou désolidarisés l'un à l'autre par vissage. Les deux éléments vissables constituent ainsi l'ouverture du réacteur permettant l'introduction d'un échantillon, non représenté, comprenant des molécules organiques à analyser.

L'élément inférieur (2) comporte un évidemment (4) destiné à recevoir l'échantillon, un épaulement circulaire (5) bordant l'évidemment (4) et une paroi latérale cylindrique (6) dont la face interne est filetée. L'élément supérieur (3) comporte une face externe cylindrique (7) qui est destinée à coopérer avec la face interne de la paroi latérale cylindrique (6) de l'élément inférieur (2) pour la solidarisation ou désolidarisation des éléments (2) et (3).

L'échantillon à analyser est introduit manuellement dans l'évidemment (4) via l'ouverture de l'élément inférieur (2) puis les éléments inférieur (2) et supérieur (3) sont solidarisés l'un à l'autre par vissage afin de former un réacteur étanche.

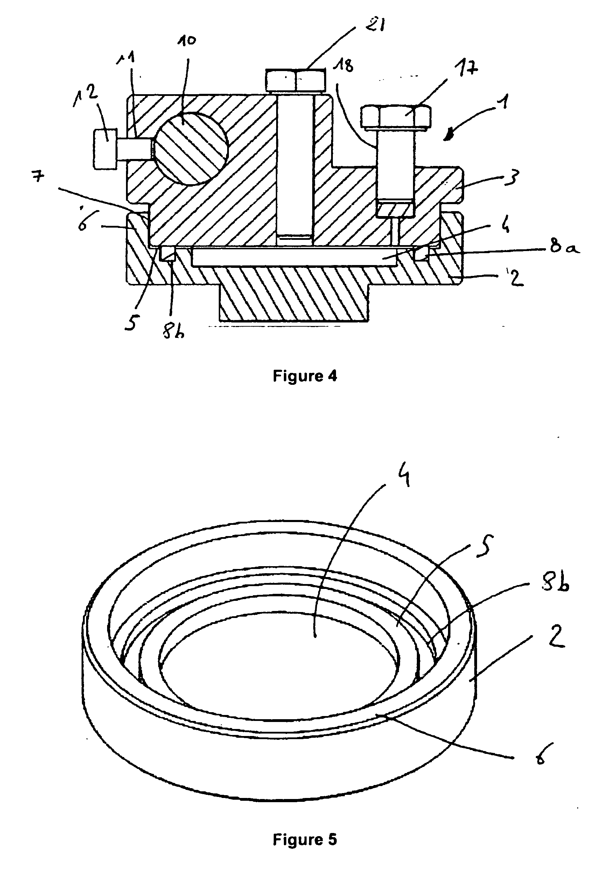

Lorsque les éléments supérieur (3) et inférieur (2) sont vissés l'un à l'autre, l'élément supérieur (3) repose sur l'épaulement circulaire (5) de l'élément inférieur. Ainsi, l'évidemment (4), obturé par l'élément supérieur (3), forme une enceinte étanche dont le volume est de 4 mL. Afin d'assurer l'étanchéité du réacteur, un joint d'étanchéité (8a) est disposé entre les éléments supérieur (3) et inférieur (2). Dans le mode de réalisation illustré (voir figure 4), une gorge (8b) annulaire de réception du joint d'étanchéité (8a) est formé dans l'épaulement (5) de l'élément inférieur (3). Le joint 8a est réalisé en graphite.

Le réacteur est de forme cylindrique et est réalisé en titane. Plus-précisément, les éléments (2), (3), (4), (5), (6) et (7) sont en titane.

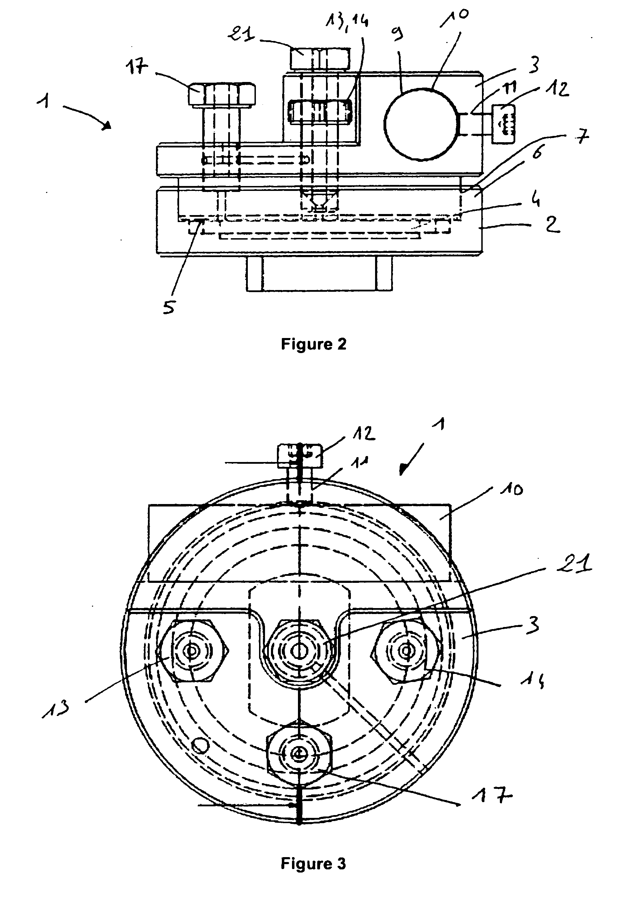

Un logement de réception (9) du moyen de chauffage (10), représenté sur les figures 2 à 4, est formé dans l'élément supérieur (3) du réacteur. Ledit logement (9) est un alésage horizontal présentant une forme cylindrique, agencé pour recevoir une cartouche chauffante amovible (10). Un orifice fileté (11) débouchant dans le logement (9) est formé dans l'élément supérieur (3). L'orifice (11) permet l'introduction d'une vis filetée (12) dont l'extrémité est destinée à coopérer avec la cartouche chauffante (10) afin de la maintenir en position dans le logement (9).

La cartouche chauffante (10) est composée d'une résistance électrique de forme cylindrique protégé par un blindage, en acier inoxydable. La puissance de la cartouche chauffante est supérieure à 400 W, permettant une montée rapide de la température du réacteur.

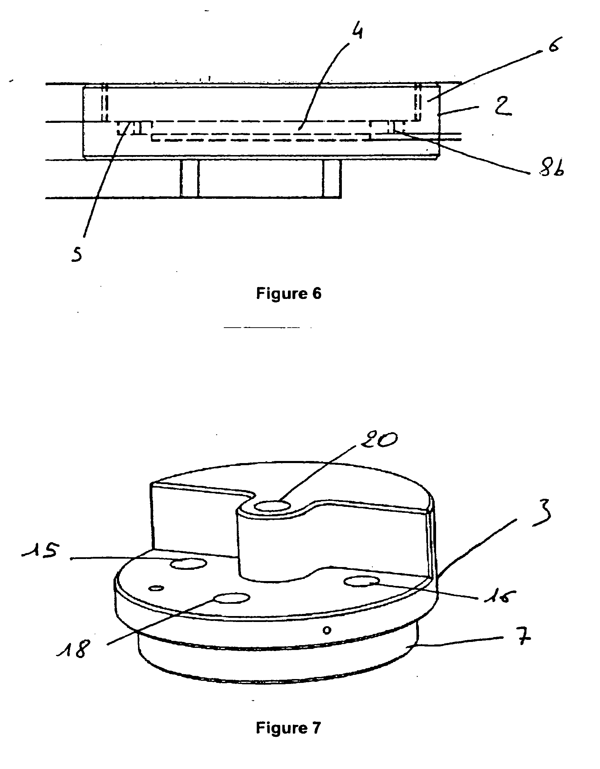

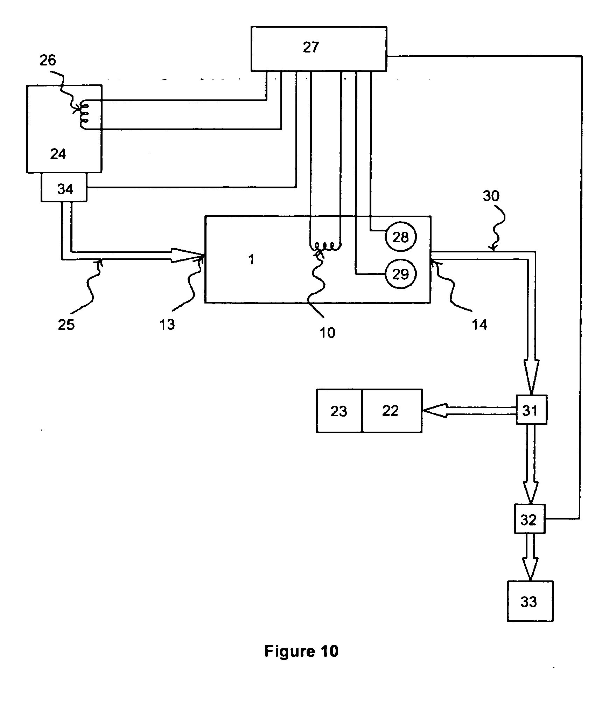

Par ailleurs, le moyen de chauffage (10) est connecté à une unité de commande (27) (voir figure 10). En outre, le dispositif (1) comprend un capteur de température (28), du type thermocouple, connecté à l'unité de commande (27) afin de réguler la température dans l'enceinte du réacteur. Le capteur de température (28) est agencé pour mesurer précisément la température, au dixième ou au centième de degrés. Il est localisé dans l'élément supérieure (3) du DPE dans un orifice filetée (20) (figure 7).

Le dispositif (1) comprend également un capteur de pression (29) à l'intérieur de l'enceinte du réacteur. Le capteur de pression sert notamment d'indicateur de la volatilisation des molécules organiques contenues dans l'échantillon. Dans le mode de réalisation représenté sur les figures 1 à 4, une vis centrale (21) coopère avec un orifice filetée (20) débouchant dans l'enceinte du réacteur. La vis centrale (21) comprend un alésage central permettant l'introduction du capteur de pression (29) ou la réalisation d'une prise de pression. Le capteur de pression (29) est également connecté à l'unité de commande (27).

Le dispositif de préparation d'échantillon (1) est également équipé d'un moyen de refroidissement (35) dudit réacteur à une vitesse moyenne de 10 à 50 °C/seconde. Dans un mode de réalisation, le moyen de refroidissement (35) est composé d'un bain d'eau apte à recevoir le réacteur.

Par ailleurs, le dispositif de préparation d'échantillon (1) comprend une entrée (13) et une sortie (14) étanches, connectées audit réacteur, et permettant la circulation d'un fluide de balayage au travers dudit réacteur. À cet effet, l'élément supérieur comprend deux orifices (15), (16) filetés débouchant à l'intérieur de l'enceinte du réacteur, à deux extrémité de celle-ci. Chacun desdits orifices (15), (16) coopère avec une vis filetée à tête hexagonale. Lesdites vis filetées sont pourvues d'un alésage central permettant le passage du fluide de balayage et sont respectivement destinées à coopérer avec une canalisation d'amenée (25) du fluide de balayage et une canalisation d'évacuation (30) dudit fluide de balayage.

En outre, le dispositif de préparation d'échantillon comprend un moyen étanche d'introduction d'une substance liquide. Dans le mode de réalisation représenté sur les figures 1 à 4, ledit moyen d'introduction de la substance liquide comprend un orifice fileté (18), formée dans l'élément supérieur du réacteur et débouchant à l'intérieur de l'enceinte, et une vis filetée (17) à tête hexagonale, munie d'un septum (19). La vis filetée (17), pourvue d'un alésage central et munie du septum (19) est introduite dans ledit orifice (18). Ainsi, la substance liquide peut être injectée au moyen d'une aiguille apte à perforer le septum, par exemple une seringue contenant ladite substance liquide.

Afin de faciliter l'extraction des molécules organiques contenues dans l'échantillon à faible température et sur un temps court, le dispositif de préparation d'échantillon est équipé d'un moyen d'agitation dans le réacteur. À cet effet, dans le présent mode de réalisation, le dispositif de préparation d'échantillon comprend une cuve à ultrasons dans laquelle le réacteur peut être placé.

L'invention est décrite dans ce qui précède à titre d'exemple. Il est entendu que l'homme du métier est à même de réaliser différentes variantes de réalisation de l'invention sans pour autant sortir du cadre de l'invention.

On peut noter que le dispositif de l'invention ne se limite pas à un réacteur en deux parties et l'on peut prévoir d'autres types d'ouvertures permettant l'introduction de l'échantillon à l'intérieur du réacteur. On peut notamment prévoir une ouverture formée dans le corps d'un réacteur monobloc, équipée d'un moyen amovible de fermeture. En outre, dans un mode de réalisation non illustré, l'ouverture est équipée d'un sas permettent le passage de l'échantillon entre le milieu extérieur et l'enceinte du réacteur. Ce mode de réalisation est particulièrement avantageux dans le cadre d'une utilisation spatiale.

Par ailleurs, on peut également prévoir un dispositif automatique d'introduction de l'échantillon à l'intérieur de l'enceinte.

### Exemple 2 : Dispositif de Préparation d'Echantillon couplé à une plaque de refroidissement.

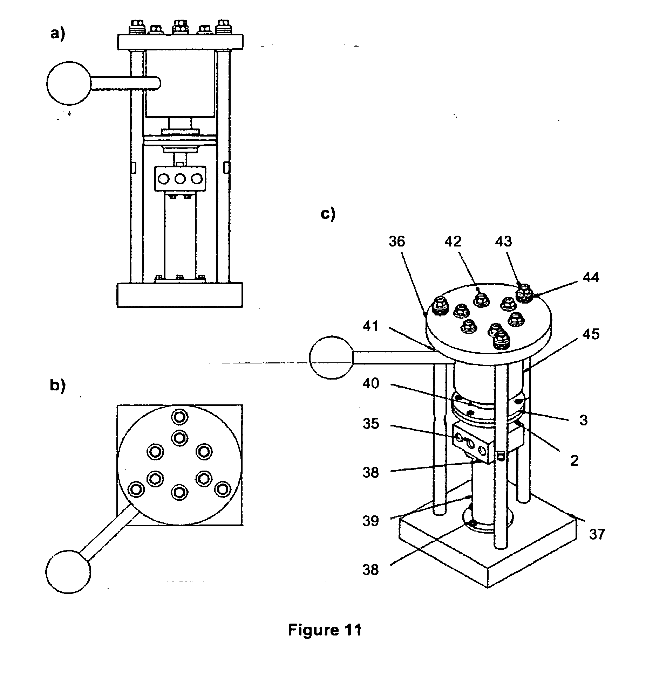

Un autre DPE a été réalisé et comporte les caractéristiques et éléments du DPE de l'exemple 1 mais avec un moyen de refroidissement différent. Dans ce second mode de réalisation, le moyen de refroidissement (35) dudit réacteur à une vitesse moyenne de 10 à 50 °C/seconde est une plaque de refroidissement en Inox (60x60x20 mm) contactée au réacteur au moyen d'une presse (Figure 11). En outre, cette plaque permet une descente en température jusqu'à -50°C grâce à une circulation d'un flux froid dans une double enveloppe ou grâce à un flux d'air froid (CO₂).

### Exemple 3: Système d'analyse comprenant un Dispositif de Préparation d'Echantillon selon l'invention

Un système d'analyse selon l'invention a été réalisé avec le DPE de l'exemple 1 et est représenté sur la figure 10.

Le système d'analyse comprend le dispositif de préparation d'échantillon (1), selon l'invention, connecté à un chromatographe en phase gazeuse (22), couplé à un spectromètre de masse (23).

Afin de permettre l'acheminement des molécules organiques volatilisées à analyser vers l'instrument d'analyse (22), le système est équipé d'un circuit de balayage comprenant un réservoir (24) de gaz inerte, une première canalisation (25) connectant le réservoir (24) à l'entrée (13) du dispositif de préparation d'échantillon (1) via une vanne (34) et une seconde canalisation (30) connectant la sortie (14) du DPE (1) à l'instrument d'analyse (22).

Le réservoir (24) est équipé d'un moyen de chauffage (26) du gaz inerte connecté à l'unité de commande (27).

Par ailleurs, le système d'analyse est équipé d'un moyen permettant le nettoyage et la vidange du dispositif. À cet effet, la canalisation de sortie (30) est équipée d'une vanne (31) connectée à l'unité de commande (27) et permettant de basculer le flux, soit vers l'entrée de l'instrument d'analyse (22), soit vers une pompe (32) dirigeant le flux vers un récipient d'évacuation (32). Ainsi, afin de nettoyer le réacteur par balayage après analyse ou d'évacuer les solvants évaporés après l'extraction, un gaz inerte est injecté via la première canalisation (25) puis aspiré par la pompe (32) afin d'évacuer les impuretés vers le récipient d'évacuation (33).

En outre, la pompe (32) connectée à l'unité de commande (27) permet de réguler la pression à l'intérieur de l'enceinte du réacteur et permet notamment de faire le vide à l'intérieur de celle-ci.

Le DPE et le système d'analyse sont illustrés par les figures 1 à 11, sur lesquelles la signification des numéros est la suivante :
1 : dispositif de préparation de l'échantillon (DPE)
2 : élément inférieur du DPE
3 : élément supérieur du DPE
4 : évidemment de l'élément inférieur (2)
5 : épaulement circulaire (5) bordant l'évidemment (4)
6 : paroi latérale cylindrique
7 : face externe cylindrique de l'élément supérieur (3)
8a : joint d'étanchéité
8b : gorge annulaire de réception du joint d'étanchéité (8)
9 : logement de réception du moyen de chauffage (10)
10 : cartouche chauffante amovible
11 : orifice fileté débouchant dans le logement (9)
12 : vis filetée introduite dans l'orifice (11) destinée à maintenir la cartouche chauffante (10)
13 : entrée du DPE connectée au réservoir (24) de gaz inerte par une canalisation (25) munie d'une vanne (34)
14 : sortie du DPE connectée à une évacuation (30), le flux gazeux pouvant être ensuite redirigé soit vers l'instrument d'analyse (22), soit vers un récipient d'évacuation (32).
15, 16 : orifices filetés débouchant à l'intérieur de l'enceinte du réacteur coopérant chacun avec une vis filetée à tête hexagonale
17 : vis filetée (17) à tête hexagonale introduite dans l'orifice (18)
18 : orifice fileté (18) débouchant à l'intérieur de l'enceinte
19 : septum coopérant avec l'orifice fileté (18) et la vis filetée (17)
20 : orifice fileté débouchant dans l'enceinte du réacteur
21 : vis centrale coopérant avec un orifice filetée (20) et comprenant un alésage central permettant l'introduction du capteur de pression (29)
22 : chromatographe en phase gazeuse
23 : spectromètre de masse
24 : réservoir de gaz inerte
25 : canalisation d'amenée du gaz inerte
27 : unité de commande
28 : capteur de température, du type thermocouple
29 : capteur de pression connecté à l'unité de commande (27)
30 : canalisation d'évacuation du gaz inerte munie d'une vanne (31)
31 : vanne de la canalisation (30) connectée à l'unité de commande (27) permettant de basculer le flux gazeux soit vers l'entrée de l'instrument d'analyse (22), soit vers une pompe (32) dirigeant le flux vers un récipient d'évacuation
32 : pompe permettant d'évacuer le flux gazeux vers un récipient d'évacuation
33 : récipient d'évacuation
34 : vanne de la canalisation (25) reliant l'entrée du DPE (13) au réservoir de gaz inerte (24)
35 : moyen de refroidissement (par exemple, plaque froide ou bain d'eau)
36 : élément supérieur de la presse
37 : élément inférieur de la presse
38 : vis
39 : support de la plaque
40 : rotor
41 : tige support
42 : vis
43 : vis
44 : rondelles Belleville.
45 : Vérin de mise en pression

### Exemple 4: Exemple de fabrication du DPE

Dans cet exemple, le DPE (1) a été réalisé par moulage. Le métal en fusion est coulé pour remplir deux moules l'un ayant la forme de l'élément inférieur (2) du DPE et l'autre ayant la forme de l'élément supérieur (3) du DPE. Dans cet exemple, le DPE est fabriqué à partir d'inox 316L ou de titane selon un usinage traditionnel à pression atmosphérique (c'est-à-dire à une pression de 100 kPa +/- 5 kPa) et à température ambiante (c'est-à-dire à une température allant de 20 à 25°C).

### Exemple 5 : Exemple d'application du DPE : réalisation du procédé « un lieu - deux étapes » (ou « one pot - two steps »)

Dans cet exemple, un procédé d'extraction, de fonctionnalisation et de volatilisation de molécules organiques contenues dans un échantillon solide est réalisé à l'aide du DPE de l'exemple 1.

L'analyse.d'un échantillon de sol du désert d'Atacama au Chili (analogue de sol martien) est effectuée suivant le protocole « un lieu - deux étapes » décrit ci-dessous.

En outre, l'utilisation d'un sol parfaitement caractérisé (sol Atacama) sert ici de référence pour comparer les performances du DPE avec celles des techniques d'extraction classiques de laboratoire. L'objectif est de démontrer la faisabilité de l'extraction et de la fonctionnalisation des molécules organiques contenues à l'état de traces dans un échantillon de sol. Le bon fonctionnement du DPE est évalué par l'analyse CPG-SM en aval.

### Protocole :

Les étapes E1 à E6 suivantes sont réalisées successivement.

### E1. Nettoyage et conditionnement du DPE :

Un état de propreté en deçà de la limite de sensibilité du CPG-SM a été établie à l'intérieur du réacteur du DPE afin de s'assurer que le seuil de résidus présents dans le DPE ne perturbera pas la détection chimique réalisée par le CPG-SM.

La limite de propreté est estimée à 10⁻¹⁵ mole pour un volume du DPE de 4 mL.

Notons que cette fonction n'est pas utile si le réacteur est dans un état de propreté satisfaisant avant introduction de l'échantillon (comme c'est le cas d'une enceinte utilisable une seule fois par exemple).

Le conditionnement est réalisé simultanément par chauffage et balayage par un gaz inerte de l'intérieur du DPE comme suit :
- la température du réacteur est élevée de 20°C (température du laboratoire) à 500°C en 15 secondes au moyen de la cartouche chauffante (10).
- la température du réacteur est maintenue à 500°C et un balayage par de l'hélium est effectué pendant 10 minutes au moyen de l'entrée (13) et de la sortie (14) permettant la circulation du gaz. Ceci permet d'effectuer une pyrolyse et d'éliminer simultanément les résidus de pyrolyse par entraînement au moyen du gaz inerte. Les caractéristiques du balayage sont les suivantes :

| **Critères** | **Caractéristiques** |
|---|---|
| Gaz inerte | hélium |
| Caractéristiques de propreté du gaz | Alpha 2 |
| Débit de gaz | 10 ml/min |
| Stabilité du débit | ± 10 % (afin d'éliminer les résidus de pyrolyse) |
| Température du gaz à l'entrée | 500°C (afin d'éviter la chute de la température de pyrolyse) |
| Caractéristiques de l'entrée de gaz | 10 mL/min |
| Caractéristiques de la sortie de gaz | 10 mL/min |
| Espace balayé | Intérieur du réacteur |
| Durée du balayage | 10 min |
| Caractéristiques physiques du dispositif d'introduction du gaz | Raccord standard étanche |
| Caractéristiques physiques du dispositif de rejet du gaz | Raccord standard étanche sans filtrage |

Afin de vérifier la propreté de l'appareil après la pyrolyse et la non présence de résidus ou de molécules organiques dans le réacteur, un test à vide peut être effectué. Ce test consiste à exécuter les étapes E3 à E6 décrites ci-après mais sans échantillon.

Les conditions de propreté requises au cours des différentes opérations suivantes sont données par la limite de détection du CPG-MS qui est de 10⁻¹⁵ mole.

Par ailleurs, les conditions de propreté requises pour l'analyse d'échantillons requièrent d'avoir un dispositif d'introduction d'échantillon respectant ce critère de propreté de 10⁻¹⁵ mole. Dans cet exemple, l'étape suivante d'introduction d'échantillon a été réalisée manuellement sous hotte à flux laminaire.

### E2. Introduction de l'échantillon :

L'échantillon à analyser est introduit manuellement, à l'aide d'une spatule métallique, au centre de l'évidemment (4) de l'élément inférieur (2) de façon à recouvrir la totalité de la surface de ce dernier. Cela permet de maximiser les surfaces de contact entre l'échantillon et les réactifs. Les caractéristiques de l'échantillon (Atacama, chili) sont les suivantes:

| **Critères** | **Caractéristiques** |
|---|---|
| Composition chimique de l'échantillon | sable contenant des molécules organiques à l'état de trace |
| Granulométrie de l'échantillon | 100 à 250µm |
| Masse de l'échantillon | 0,5 g |
| Volume de l'échantillon | environ 0,3 mL |
| Propriétés et forme de l'échantillon | Solide et sec |
| Caractéristiques du lieu de dépôt de l'échantillon au sein du dispositif | - Dépôt uniforme pour assurer une bonne mouillabilité |
| | - Reproductibilité du dépôt |
| Condition de propreté requise au cours de cette opération | - Limite de détection du CPG-MS : 10⁻¹⁵ mole |
| | - Recours à un dispositif d'introduction respectant ce critère |
| Température du réacteur en début d'introduction | Ambiante (25 °C) |

Une fois l'échantillon introduit, le réacteur est refermé. La température et la pression de l'échantillon sont contrôlées et sont respectivement de 25°C et de 1 bar.

### E3. Extraction à l'aide d'un solvant :

Le solvant d'extraction est introduit à température ambiante dans le réacteur au moyen d'une seringue par l'entrée (17) munie d'un septum. Les caractéristiques du solvant sont les suivantes :

| Nature du solvant | Eau - Isopropanol (1 :1) |
|---|---|
| Volume | 1 mL (soit 0,5 mL d'eau et 0,5 mL d'isopropanol) |

L'agitation est ensuite effectuée afin de permettre l'extraction des molécules organiques contenues dans l'échantillon.

Ici, l'agitation a été effectuée par immersion du réacteur dans une cuve à ultrasons. Les caractéristiques de l'agitation sont les suivantes :

| Caractéristiques de l'émission des ultrasons | HF. Fréquence : 35 kHz (2.4 A, 230 V, 50/60 Hz) - AC- |
|---|---|
| Durée de l'agitation | 30 min |
| Température lors de l'agitation | 60°C |

### E4. Elimination du solvant d'extraction :

Le solvant est éliminé par évaporation à l'aide d'un balayage de l'enceinte par un gaz à une température modérée au moyen de l'entrée (13) et de la sortie (14) permettant la circulation du gaz. On peut noter que les molécules organiques à analyser ne sont pas entraînées au cours du balayage compte tenu du chauffage modéré d'une part et de leur propriété réfractaire, c'est-à-dire non volatile d'autre part. L'ouverture et la fermeture de l'orifice d'évacuation du gaz sont commandées manuellement. Les caractéristiques du balayage sont les suivantes :

| Température du gaz fourni en entrée | 30 °C (pour éviter l'évaporation des molécules organiques volatiles) |
|---|---|
| Gaz utilisé pour le balayage | Hélium |
| Propreté du gaz | Alpha 2 |
| Débit du gaz | 1 mL/min |
| Durée du balayage et du maintien en température | 15 min |
| Température intérieure du DPE | 30°C |

Eventuellement, une grille de protection placée en sortie du gaz permet de conserver l'échantillon à l'intérieur du réacteur.

### E5. Fonctionnalisation des molécules organiques extraites :

Une solution comprenant un agent de fonctionnalisation est ensuite introduite dans le réacteur afin de fonctionnaliser les molécules organiques extraites et de les rendre volatiles. L'agent de fonctionnalisation est introduit dans le réacteur, après que la température de celui-ci soit ramenée à 20°C. L'introduction de l'agent de fonctionnalisation est effectuée au moyen d'une seringue par l'entrée (17) munie du septum (19).

La solution utilisée ici est une solution de MTBSTFA dans le DMF obtenue en mélangeant 30 µL de MTBSTFA commercialisé sous la référence 19915 par la société Sigma-Aldrich, France et 10 µL de DMF commercialisé sous la référence 227056 par la société Sigma-Aldrich, France.

Les caractéristiques et quantités de solution d'agent de fonctionnalisation introduites sont les suivantes :

| Solution d'agent de fonctionnalisation | Une solution de MTBSTFA pur (30 µL) dans le DMF pur (10 µL) |
|---|---|
| Volume de solution introduit | 40 µL |

La température du réacteur est ensuite élevée à la température T_{f} de 75°C +/- 5°C en une durée de 3 minutes +/- 1 minute.

Puis, la température T_{f} est maintenue pendant une durée de 15 minutes +/- 1 minute afin de réaliser la réaction de fonctionnalisation des molécules organiques extraites.

La température à l'intérieur de l'enceinte est régulée à l'aide de moyens de contrôle et de commande de la température qui permettent de mesurer la température à l'intérieur de l'enceinte avec une précision de +/-1°C et d'ajuster la température.

### E6. Volatilisation des molécules organiques fonctionnalisées :

La température du réacteur est ensuite élevée à la température Tᵥ de 200°C +/- 5°C en une durée de 15 secondes +/- 10 secondes au moyen de la cartouche chauffante (10). La température à l'intérieur de l'enceinte est régulée à l'aide de moyens de contrôle et de commande.

Puis, la température Tᵥ est maintenue pendant une durée de 10 minutes afin de réaliser la volatilisation des molécules organiques.

Simultanément, un balayage de l'enceinte par un gaz inerte est effectué au moyen de l'entrée (13) et de la sortie (14) permettant la circulation du gaz afin de transférer les molécules organiques volatilisées vers un instrument d'analyse CPG-SM (22) et (23). Les caractéristiques du balayage sont les suivantes :

| Gaz utilisé pour le balayage (gaz pousseur) | | Hélium alpha 2 ou autre gaz inerte |
|---|---|---|
| Caractéristique du flux en entrée | | |
| | - balayage : | Balayage continu |
| | - débit | 1 mL/min |
| Caractéristique du flux à l'intérieur | | Balayage continu |
| Caractéristique de l'écoulement | | |
| | - type de flux | Laminaire |
| | - débit | 1 mL/min |
| | - pression | 1 bar |
| | - volume | 4 mL |

Eventuellement, une grille de protection placée en sortie du gaz permet de conserver l'échantillon à l'intérieur du réacteur.

On peut noter que toutes les molécules volatilisées présentes dans le réacteur, qu'elles soient fonctionnalisées ou non, peuvent être transférées vers l'instrument d'analyse. Par exemple, le solvant ou l'agent de fonctionnalisation restants utilisé au cours de l'étape de fonctionnalisation peut être volatilisé en même temps que les molécules organiques fonctionnalisées et transférée.

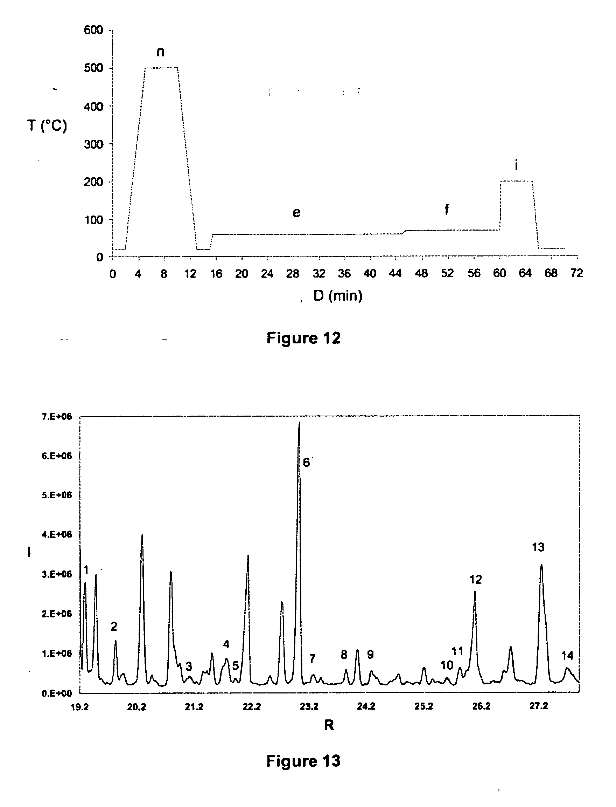

L'ensemble des étapes de cet exemple peut être illustré par la figure 12 qui représente le chronogramme synthétisant les différents cycles de température T (en °C) en fonction de la durée D (en minute) de l'expérience pour l'expérimentation « un lieu / deux étapes » (ou « one pot / two steps »). Les étapes (n), (e), (f) et (i) représentent respectivement les étapes de nettoyage du réacteur, d'extraction des molécules organiques, de fonctionnalisation des molécules organiques extraites, et d'injection des molécules organiques volatilisées dans l'instrument d'analyse.

### Résultats :

La figure 13 représente le chromatogramme issu de l'analyse chromatographique couplé à la spectrométrie de masse de l'échantillon de 0,5 g de sol du désert d'Atacama après le procédé « un lieu / deux étapes » (ou « one pot / two steps »), c'est-à-dire après extraction aux ultrasons (30 minutes) par un mélange de 0,5 mL d'isopropanol et de 0,5 mL d'eau, évaporation du solvant sous flux d'hélium, traitement par le mélange MTBSTFA / DMF (75°C, 15 minutes) et volatilisation.

Le chromatogramme de la figure 13 représente l'évolution de l'intensité I du signal du détecteur, qui est lié à la concentration instantanée de la molécule organique éluée, en fonction du temps de rétention R (en minute). Sur cette représentation graphique, des pics numérotés émergent et correspondent aux molécules organiques, fonctionnalisée par le MTBSTFA, suivantes : 1 : acide benzoïque, 2 : acide 2-hydroxy propanoïque, 3: alanine, 4 : acide 2-hydroxy butanoïque, 5 : glycine, 6: acide nonanoïque, 7: sarcosine, 8: acide 3-méthyle benzoïque, 9: acide 2-butanoïque, 10 : acide hexanoïque, 11 : valine, 12 : urée, 13 : acide decanoïque, 14 : norleucine.

En conclusion, le tableau suivant présente tous les composés organiques détectés (D) ou non (ND) dans l'échantillon de 0,5 g de sol d'Atacama (Chili) en utilisant la technique « one pot - two steps ».

**Tableau 2**

| **Composés organiques contenu dans le sol du désert d'Atacama (Chili)** | **Procédure « one pot - two steps »** |
|---|---|
| Acide benzoïque | D |
| Acide acétique | D |
| Alanine | D |
| Glycine | D |
| Acide sulphurique | D |
| Acide nonanoïque | D |
| Valine | D |
| Leucine | D |
| Proline | D |
| Acide 1,2-benzendicarboxylique | D |
| Acide Phosphorique | D |
| Benzendicarboxylate bis(2-methylpropyl) | D |
| Serine | D |
| Phenylalanine | D |
| Acide dodécanoïque | ND |
| Acide tétradécanoïque | D |
| Acide pentadécanoïque | ND |
| Acide glutamique | D |
| Acide octadecanoïque | D |
| Benzendicarboxylate bis(2-methylpropyl) | D |

Les résultats qualitatifs obtenus à partir de la procédure "one pot - two steps" ont permis de retrouver la majorité des composés contenus dans le sol d'Atacama.

### Exemple 6 : Exemple d'application du DPE : réalisation du procédé « un lieu - une étape » (ou « one pot - one step »)

Dans cet exemple, un procédé d'extraction, de fonctionnalisation et de volatilisation de molécules organiques contenues dans un échantillon solide est réalisé à l'aide du DPE de l'exemple 1.

L'analyse d'un échantillon de sol du désert d'Atacama au Chili (analogue de sol martien) est effectuée suivant le protocole « one pot - one step » décrit ci-dessous.

L'utilisation d'un sol parfaitement caractérisé (sol Atacama) sert ici de référence pour comparer les performances du DPE avec celles des techniques d'extraction classique de laboratoire. L'objectif est de démontrer la faisabilité de l'extraction et de la fonctionnalisation des molécules organiques contenues à l'état de traces dans un échantillon de sol. Le bon fonctionnement du DPE sera évalué par l'analyse CPG-SM en aval.

### Protocole :

Les étapes E1 à E6 suivantes sont réalisées successivement.

### E1. Nettoyage et conditionnement du DPE :

Un état de propreté en deçà de la limite de sensibilité du CPG-SM a été établie à l'intérieur du réacteur du DPE afin de s'assurer que le seuil de résidus présents dans le DPE ne perturbera pas la détection chimique réalisée par le CPG-SM.

La limite de propreté est estimée à 10⁻¹⁵ mole pour un volume du DPE de 4 mL.

Notons que cette fonction n'est pas utile si le réacteur est dans un état de propreté satisfaisant avant introduction de l'échantillon (comme c'est le cas d'une enceinte utilisable une seule fois par exemple).

Le conditionnement est réalisé simultanément par chauffage et balayage par un gaz inerte de l'intérieur du DPE comme suit :
- la température du réacteur est élevée de 20°C (température du laboratoire) à 500°C en 15 secondes au moyen de la cartouche chauffante (10).
- la température du réacteur est maintenue à 500°C et un balayage par de l'hélium est effectué pendant 10 minutes afin d'effectuer une pyrolyse et d'éliminer les résidus de pyrolyse par entraînement au moyen du gaz inerte. Les caractéristiques du balayage sont données dans le tableau suivant :

| **Critères** | **Caractéristiques** |
|---|---|
| Gaz inerte | Hélium |
| Caractéristiques de propreté du gaz | Alpha 2 |
| Débit gaz | 10 ml/min |
| Stabilité du débit | ± 10 % (afin d'éliminer les résidus de pyrolyse) |
| Température du gaz à l'entrée | 500°C (afin d'éviter la chute de la température de pyrolyse) |
| Caractéristiques de l'entrée de gaz | 10 mL/min |
| Caractéristiques de la sortie de gaz | 10 mL/min |
| Espace balayé | Intérieur du réacteur |
| Durée du balayage | 10 min |
| Caractéristiques physiques du dispositif d'introduction du gaz | Raccord standard étanche |
| Caractéristiques physiques du dispositif de rejet du gaz | Raccord standard étanche sans filtrage |

Afin de vérifier la propreté de l'appareil après la pyrolyse et la non-présence de résidus ou de molécules organiques dans le réacteur, un test à vide peut être effectué. Ce test consiste à exécuter les étapes E3 à E6 décrites ci-après mais sans échantillon.

Les conditions de propreté requises au cours des différentes opérations suivantes sont données par la limite de détection du CPG-MS qui est de 10⁻¹⁵ mole.

Par ailleurs, les conditions de propreté requises pour l'analyse d'échantillons nécessitent d'avoir un dispositif d'introduction d'échantillon respectant ce critère de propreté de 10⁻¹⁵ mole. Dans cet exemple, l'étape suivante d'introduction d'échantillon a été réalisée manuellement sous une hotte à flux laminaire.

### E2. Introduction de l'échantillon :

L'échantillon à analyser est introduit manuellement, à l'aide d'une spatule métallique, au centre de l'évidemment (4) de l'élément inférieur (2) de façon à recouvrir la totalité de la surface de ce dernier. Cela permet de maximiser les surfaces de contact entre l'échantillon et les réactifs. Les caractéristiques de l'échantillon sont les suivantes:

| **Critères** | **Caractéristiques** |
|---|---|
| Composition chimique de l'échantillon | sable contenant des molécules organiques à l'état de trace |
| Granulométrie de l'échantillon | 100 à 250µm |
| Masse de l'échantillon | 0,5 g |
| Volume de l'échantillon | environ 0,3 mL |
| Propriétés et forme de l'échantillon | Solide et sec |
| Caractéristiques du lieu de dépôt de l'échantillon au sein du dispositif | - Dépôt uniforme pour assurer une bonne mouillabilité |
| | - Reproductibilité du dépôt |
| Condition de propreté requise au cours de cette opération | - Limite de détection du CPG-MS : 10⁻¹⁵ mole |
| | - Recours à un dispositif d'introduction respectant ce critère |
| Température du réacteur en début d'introduction | Ambiante (25 °C) |

Une fois l'échantillon introduit, le réacteur est refermé. La température et la pression de l'échantillon sont contrôlées et sont respectivement de 25°C et de 1 bar.

### E3. Extraction par thermo-désorption :

### Mise sous pression de l'échantillon

Après l'introduction de l'échantillon dans l'enceinte du réacteur, une pression de 4 mbar +/- 3 mbar est appliquée.

### Elévation de la température de l'échantillon

La température du réacteur est ensuite élevée à la température T_{d} de 500°C +/- 5°C en 15 secondes au moyen de la cartouche chauffante (10).

### Maintient de la température

La température du réacteur est ensuite maintenue à la température T_{d} de 500°C +/- 5°C pendant 5 minutes afin de désorber par thermo-désorption les molécules organiques contenues dans l'échantillon solide.

On peut noter que la température à l'intérieur de l'enceinte est régulée à l'aide de moyens de contrôle et de commande de température.

### E4. Conditionnement du réacteur du DPE pour la fonctionnalisation des molécules organiques extraites :

### Diminution de la température

Un refroidissement est effectué afin d'obtenir une descente rapide de la température. Dans le cadre de cet exemple, la température est diminuée en 15 secondes de la température T_{d} à la température T_{f} de 75°C +/- 1°C à l'aide du moyen de refroidissement (35). Dans une réalisation, le moyen de refroidissement est un bain d'eau froide, dans une autre réalisation, le moyen de refroidissement est une plaque froide telle que décrite dans l'exemple 2.

### Maintient de la température

L'étape de diminution de la température est suivie d'une étape de stabilisation de la température afin de laisser l'échantillon atteindre la température T_{f} pour la réaction de fonctionnalisation. Ici, la température T_{f} est maintenue pendant une durée de 5 minutes.

La température à l'intérieur de l'enceinte est régulée à l'aide de moyens de contrôle et de commande de température.

### E5. Fonctionnalisation des molécules organiques extraites :

Une solution comprenant un agent de fonctionnalisation à température ambiante (20 °C) est introduite dans le réacteur au moyen d'une seringue par l'entrée (17) munie d'un septum (19).

La solution utilisée ici est obtenue en mélangeant 30 µL de MTBSTFA commercialisé sous la référence 19915 par la société Sigma-Aldrich (France) et 10 µL de DMF commercialisé sous la référence 227056 par la société Sigma-Aldrich (France).

Les caractéristiques et quantités de solution d'agent de fonctionnalisation introduites sont les suivantes :

| Solution d'agent de fonctionnalisation | Une solution de MTBSTFA pur (30 µL) dans le DMF pur (10 µL) |
|---|---|
| Volume total de solution introduit | 40 µL |

La température du réacteur est ensuite maintenue à la température T_{f} de 75°C +/- 5°C.

Puis, la température T_{f} est maintenue pendant une durée de 30 minutes +/- 1 minute afin de réaliser la réaction de fonctionnalisation des molécules organiques extraites.

On peut noter que la température à l'intérieur de l'enceinte est régulée à l'aide de moyens de contrôle et de commande de température qui permettent de mesurer la température à l'intérieur de l'enceinte avec une précision de +/-1°C et d'ajuster la température.

### E6. Volatilisation des molécules organiques fonctionnalisées :

La température du réacteur est ensuite élevée à la température Tᵥ de 200°C +/- 5°C en une durée de 15 secondes +/- 10 secondes au moyen de la cartouche chauffante (10). La température à l'intérieur de l'enceinte est régulée à l'aide de moyens de contrôle et de commande de température.

Puis, la température Tᵥ est maintenue pendant une durée de 10 minutes afin de réaliser la volatilisation des molécules organiques. [00218] Simultanément, un balayage de l'enceinte par un gaz inerte est effectué au moyen de l'entrée (13) et de la sortie (14) permettant la circulation du gaz afin de transférer les molécules organiques volatilisées vers un instrument d'analyse CPG-SM. Les caractéristiques du balayage sont les suivantes :

| Gaz inerte utilisé pour le balayage (gaz pousseur) | | Hélium alpha 2 |
|---|---|---|
| Caractéristique du flux en entrée | | |
| | - balayage : | Balayage continu |
| | - débit | 1 mL/min |
| Caractéristique du flux à l'intérieur | | Balayage continu |
| | Caractéristique de l'écoulement | |
| | - type de flux | Laminaire |
| | - débit | 1 mL/min |
| | - pression | 1 bar |
| | - volume | 1 mL |

Eventuellement, une grille de protection placée en sortie du gaz permet de conserver l'échantillon à l'intérieur du réacteur.

On peut noter que toutes les molécules volatilisées présentes dans le réacteur, qu'elles soient fonctionnalisées ou non, peuvent être transférées vers l'instrument d'analyse. Par exemple, les restes de solvant et/ou d'agent de fonctionnalisation utilisés au cours de l'étape de fonctionnalisation peuvent être volatilisés, en même temps que les molécules organiques fonctionnalisées et transférée.

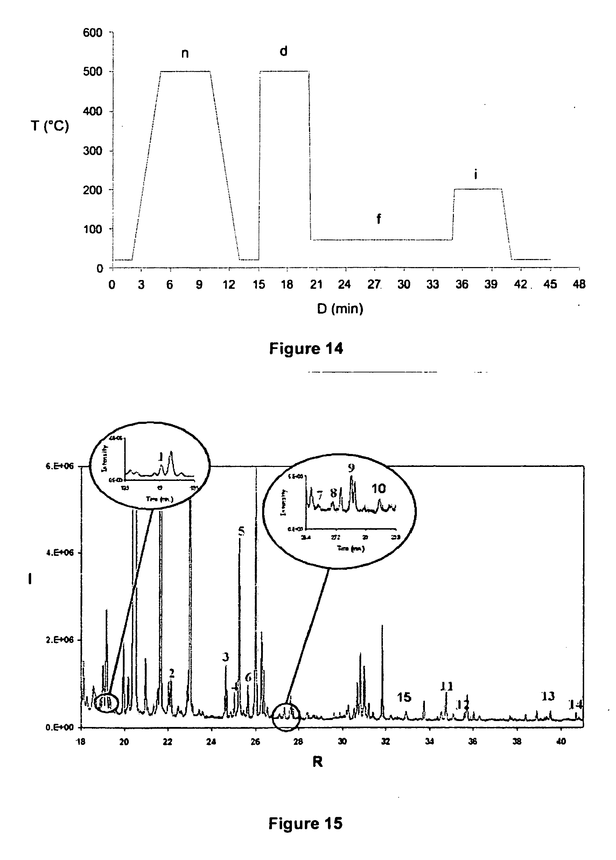

L'ensemble des étapes de cet exemple peut être illustré par la figure 14 qui représente le chronogramme synthétisant les différents cycles de température en fonction du temps de l'expérience pour l'expérimentation « un lieu / une étape » (ou « one pot / one step »). Les étapes (n), (d), (f) et (i) représentent respectivement les étapes de nettoyage du réacteur, de thermodésorption des molécules organiques, de fonctionnalisation des molécules organiques désorbées, et d'injection des molécules organiques volatilisées dans l'instrument d'analyse.

### Résultats :

La figure 15 représente le chromatogramme issu de l'analyse chromatographique couplé à la spectrométrie de masse de l'échantillon de 0,5 g de sol du désert d'Atacama après le procédé « un lieu / une étape » (ou « one pot / one step »), c'est-à-dire après chauffage à 500°C durant 5 minutes, traitement par un mélange MTBSTFA / DMF (75°C, 15 minutes) et volatilisation.

Le chromatogramme de la figure 15 représente l'évolution de l'intensité I du signal du détecteur, qui est lié à la concentration instantanée de la molécule organique éluée, en fonction du temps de rétention R (en minute). Sur cette représentation graphique, des pics numérotés émergent et correspondent aux molécules organiques, fonctionnalisée par le MTBSTFA, suivantes : 1 : acide acetique , 2 : acide propanoïque, 3: acide benzoïque, 4 : acide heptanoïque, 5 : acide octanoïque, 6: acide 4-methyl pentanoïque, 7 : alanine, 8 : glycine, 9: acide nonanoïque, 10: valine, 11 : acide dodecanoïque, 12: acide phosphorique, 13: acide 1,2 benzendicarboxylique, 14: acide pentandecanoïque, 15 : 1,2 benzendicarboxylate bis(2-méthylpropyl).

En conclusion, le tableau suivant présente tous les composés organiques détectés (D) ou non (ND) dans l'échantillon de 0,5 g de sol d'Atacama (Chili) en utilisant la technique « one pot - one step ».

**Tableau 3**

| **Composés organiques contenu dans le sol du désert d'Atacama (Chili)** | **Procédure "one pot - one step"** |
|---|---|
| Acide benzoïque | D |
| Acide acétique | D |
| Alanine | D |
| Glycine | D |
| Acide sulphurique | ND |
| Acide nonanoïque | D |
| Valine | D |
| Leucine | ND |
| Proline | ND |
| Acide 1,2-benzendicarboxylique | D |
| Acide Phosphorique | D |
| Benzendicarboxylate bis(2-methylpropyl) | D |
| Serine | ND |
| Phenylalanine | ND |
| Acide dodécanoïque | D |
| Acide tétradécanoïque | ND |
| Acide pentadécanoïque | D |
| Acide glutamique | ND |
| Acide octadécanoïque | D |
| Benzendicarboxylate bis(2-methylpropyl) | D |

Les résultats qualitatifs obtenues à partir de la procédure "one pot - one step" ont permis de retrouver la majorité des acides carboxyliques contenus dans le sol d'Atacama. Une partie des acides aminés (les plus légers) ont pu être détectés. Cette technique a l'avantage de ne nécessiter aucun solvant et d'être réalisable en une seule étape. Ces résultats montrent que le dispositif de préparation d'échantillon de l'invention permet d'extraire et de détecter plus de 65 % des molécules présentes dans l'échantillon. En outre, le procédé et le dispositif décrits dans la présente sont parfaitement adaptés à l'analyse *in situ.*

### Liste des références

(1) Dadkha Ali et al., Hot water extraction with in situ wet oxidation : PAHs removal from soil, Journal of hazardous materials, 2002, 93(3), 307-320.
(2) Mowry Curtis et al., Rapid detection of bacteria with miniaturized pyrolysis-gas chromatographic analysis, Proceeding of SPIE, 2001, Vol. 4575, pp. 83-90.
(3) A. Buch, D.P. Glavin, R. Sternberg, C. Rodier, C. Szopa, A new extraction technique for in situ analyses of amino and carboxylic acids on Mars by gas chromatography mass spectrometry, Planetary and Space Science, Special Astrobiology Issue, 54, 15, (2006), 1592-1599.
(4) Buch A., Marchetti C., Meunier D., Sternberg R., Raulin F., Solvent extraction of organic molecules of exobiological interest for in situ analysis of the Martian soil, Journal of Chromatography A. 999 (2003) 165-174.
(5) Brevet US N° 6,511,760.
(6) R. Sternberg, A. Buch, C. Szopa, C. Vidal-Madjar, F. Raulin, Gas chromatography in space exploration, Encyclopaedia of Separation Science, Academic Press, London, (Edition: I.D. Wilson, E.R. Adlard, M. Cooke, C.F. Poole) (2006).
(7) D. Meunier, R. Sternberg, A. Buch, C. Szopa, C. Rodier, M. Cabane, F. Raulin, A laboratory pilot for in situ analysis of refractory organic matter in Martian soil by gas chromatography-mass spectrometry. Advances in Space Research (2007) 39, 3, 337-344

## Revendications

1. Dispositif de préparation d'échantillon (1) comprenant :
- un réacteur étanche comportant un moyen d'introduction d'un échantillon comprenant des molécules organiques ;
- au moins une entrée (13) et une sortie (14) étanches connectées audit réacteur et permettant la circulation d'un fluide de balayage au travers dudit réacteur ;
- un moyen de commande (27) et de contrôle (28) de température dans le réacteur ;
- un moyen de contrôle (29) de la pression dans le réacteur ; et
- une sonde appliquant des ultrasons à l'intérieur du réacteur,
**caractérisé en ce qu'**il comprend en outre :
- un moyen de chauffage (10) dudit réacteur à une vitesse moyenne de 10 à 50 °C/seconde ;
- un moyen de refroidissement (35) dudit réacteur à une vitesse moyenne de 10 à 50 °C/seconde, ledit le moyen de refroidissement (35) étant une plaque de refroidissement traversée par un circuit de refroidissement, ledit moyen de refroidissement étant mis en contact avec le réacteur au moyen d'une presse.

2. Dispositif de préparation d'échantillon (1) selon la revendication 1, dans lequel le fluide de balayage est un gaz inerte.

3. Dispositif de préparation d'échantillon (1) selon la revendication 1 ou 2, comprenant en outre un moyen de chauffage (26) du fluide de balayage.

4. Dispositif de préparation d'échantillon (1) selon l'une quelconque des revendications 1 à 3 comprenant en outre un moyen (32) permettant de faire le vide ou d'appliquer une pression dans le réacteur.

5. Dispositif de préparation d'échantillon (1) selon l'une quelconque des revendications 1 à 4, comprenant en outre un moyen d'aspiration (32) de fluides ou de molécules organiques volatilisées.

6. Dispositif de préparation d'échantillon (1) selon l'une quelconque des revendications 1 à 5, comprenant en outre au moins un moyen (32, 33) de nettoyage du réacteur.

7. Dispositif de préparation d'échantillon (1) selon l'une des revendications 1 à 6, comprenant en outre au moins un moyen de rétention de l'échantillon solide dans le réacteur agencé pour retenir l'échantillon lors de la circulation du fluide de balayage.

8. Dispositif de préparation d'échantillon (1) selon l'une des revendications 1 à 7, comprenant en outre au moins un moyen étanche (17, 18) d'introduction d'une substance liquide dans le réacteur.

9. Dispositif de préparation d'échantillon (1) selon la revendication 8, comprenant en outre au moins un réservoir destiné à contenir une substance liquide, connecté audit moyen étanche d'introduction d'une substance liquide,

10. Dispositif de préparation d'échantillon (1) selon l'une quelconque des revendications 1 à 9, dans lequel le moyen de contrôle de la température est un capteur de température (28) positionné dans le réacteur.

11. Dispositif de préparation d'échantillon (1) selon l'une quelconque des revendications 1 à 10, dans lequel le moyen de contrôle de la pression comprend un capteur de pression positionné dans le réacteur.

12. Dispositif de préparation d'échantillon (1) selon l'une quelconque des revendications 1 à 11, dans lequel le réacteur est constitué d'un matériau choisi dans le groupe comprenant l'inox, le cuivre, le titane, l'acier ou la céramique.

13. Dispositif de préparation d'échantillon (1) selon l'une quelconque des revendications 1 à 12, dans lequel le volume de l'enceinte du réacteur est de 2 à 10 mL.

14. Système d'analyse **caractérisé en ce qu'**il comprend au moins un dispositif de préparation d'échantillon (1) selon l'une quelconque des revendications 1 à 13, et au moins un instrument d'analyse (22) connecté en aval dudit dispositif de préparation d'échantillon (1).

15. Système d'analyse selon la revendication 14, dans lequel l'instrument d'analyse est un chromatographe en phase gazeuse (22).

16. Système d'analyse selon la revendication 14 ou 15, dans lequel l'instrument d'analyse (22) est un chromatographe en phase gazeuse couplé à un spectromètre de masse (23) ou à un détecteur.

17. Système d'analyse selon l'une quelconque des revendications 14 à 16, comprenant en outre un circuit de balayage des molécules organiques comprenant :
- un réservoir (24) de fluide de balayage ;
- une canalisation (25) connectant ledit réservoir (24) de fluide de balayage à l'entrée étanche (13) du dispositif de préparation d'échantillon (1); et
- une canalisation (30) connectant la sortie (14) du dispositif de préparation d'échantillon (1) à l'instrument d'analyse (22).

## Patentansprüche

1. Vorrichtung zur Vorbereitung einer Probe (1) umfassend:
- Ein dichtes Reaktionsgefäß mit einem Mittel zur Einführung einer Probe, die organische Moleküle umfasst;
- mindestens einen jeweils dichten Eintritt (13) und Austritt (14), die an das Reaktionsgefäß angeschlossen sind und die das Durchströmen eines Spülmediums durch das Reaktionsgefäß ermöglichen;
- ein Mittel zur Steuerung (27) und zur Überwachung (28) der Temperatur in dem Reaktionsgefäß;
- ein Mittel zur Steuerung (29) des Drucks in dem Reaktionsgefäß; und
- eine Sonde, die im Inneren des Reaktionsgefäßes Ultraschall anwendet,
**dadurch gekennzeichnet, dass** die Vorrichtung ferner folgende umfasst:
- Ein Mittel zum Heizen (10) des Reaktionsgefäßes mit einer mittleren Geschwindigkeit von 10 bis 50 °C/Sekunde;
- Ein Mittel zum Kühlen (35) des Reaktionsgefäßes mit einer mittleren Geschwindigkeit von 10 bis 50 °C/Sekunde, wobei das Mittel zum Kühlen (35) eine Kühlplatte ist, die von einem Kühlkreislauf durchlaufen wird, wobei die Kühlmittel mit dem Reaktionsgefäß mittels einer Presse in Kontakt gebracht werden.

2. Vorrichtung zur Vorbereitung einer Probe (1) nach Anspruch 1, wobei das Spülmedium ein inertes Gas ist.

3. Vorrichtung zur Vorbereitung einer Probe (1) nach Anspruch 1 oder 2, ferner umfassend ein Mittel zum Heizen (26) des Spülmediums.

4. Vorrichtung zur Vorbereitung einer Probe (1) nach einem der Ansprüche 1 bis 3 ferner umfassend ein Mittel (32), mit dem ein Vakuum geschaffen oder Druck im Reaktionsgefäß angewendet werden kann.

5. Vorrichtung zur Vorbereitung einer Probe (1) nach einem der Ansprüche 1 bis 4, ferner umfassend ein Mittel zur Absaugung (32) von Medien oder verflüchtigten organischen Molekülen.

6. Vorrichtung zur Vorbereitung einer Probe (1) nach einem der Ansprüche 1 bis 5, ferner mindestens ein Mittel (32, 33) zur Reinigung des Reaktionsgefäßes umfassend.

7. Vorrichtung zur Vorbereitung einer Probe (1) nach einem der Ansprüche 1 bis 6, ferner ein Mittel zur Zurückhaltung der festen Probe in dem Reaktionsgefäß umfassend, das angebracht ist, um die Probe während der Strömung des Spülmediums zurückzuhalten.

8. Vorrichtung zur Vorbereitung einer Probe (1) nach einem der Ansprüche 1 bis 7, die ferner mindestens ein dichtes Mittel (17, 18) zur Einführung einer flüssigen Substanz in das Reaktionsgefäß umfasst.

9. Vorrichtung zur Vorbereitung einer Probe (1) nach Anspruch 8, die ferner einen Behälter umfasst, der die flüssige Substanz enthält, und an das dichte Mittel zur Einführung einer flüssigen Substanz angeschlossen ist.

10. Vorrichtung zur Vorbereitung einer Probe (1) nach einem der Ansprüche 1 bis 9, wobei das Temperaturüberwachungsmittel ein in dem Reaktionsgefäß angebrachter Temperatursensor (28) ist.

11. Vorrichtung zur Vorbereitung einer Probe (1) nach einem der Ansprüche 1 bis 10, wobei das Drucküberwachungsmittel einen in dem Reaktionsgefäß angebrachten Drucksensor umfasst.

12. Vorrichtung zur Vorbereitung einer Probe (1) nach einem der Ansprüche 1 bis 11, wobei das Reaktionsgefäß aus einem Material besteht, das aus der Gruppe gewählt wird, die nichtrostenden Stahl, Kupfer, Titan, Stahl oder Keramik umfasst.

13. Vorrichtung zur Vorbereitung einer Probe (1) nach einem der Ansprüche 1 bis 12, wobei das Volumen des Behälters des Reaktionsgefäßes 2 bis 10 mL beträgt.

14. Analysesystem, **dadurch gekennzeichnet, dass** es mindestens eine Vorrichtung zur Vorbereitung der Probe (1) nach einem der Ansprüche 1 bis 13 und mindestens ein Analysegerät (22) umfasst, das der Vorrichtung zur Vorbereitung der Probe (1) nachgeschaltet angeschlossen ist.

15. Analysesystem nach Anspruch 14, wobei das Analysegerät (22) ein Gaschromatograph ist.

16. Analysesystem nach Anspruch 14 oder 15, wobei das Analysegerät (22) ein mit einem Massenspektrometer oder einem Detektor gekuppelter Gaschromatograph (23) ist.

17. Analysesystem nach einem der Ansprüche 14 bis 16, das ferner einen Spülgaskreislauf für organische Moleküle umfasst mit :
- Einem Behälter (24) für das Spülmedium;
- einer Rohrleitung (25), die den Behälter (24) für das Spülmedium mit dem dichten Eintritt (13) der Vorrichtung zum Vorbereiten einer Probe (1) verbindet, und
- einer Rohrleitung (30), die den Austritt (14) der Vorrichtung zum Vorbereiten einer Probe (1) mit dem Analysegerät (22) verbindet.

## Claims

1. Sample preparation device (1) comprising:
- a sealed reactor containing means for introducing a sample comprising organic molecules;
- at least one sealed inlet (13) and one sealed outlet (14) connected to said reactor and allowing the circulation of a displacement fluid through said reactor;
- a command means (27) and a control means (28) of the temperature in the reactor;
- a control means (29) of the pressure in the reactor, and
- a probe applying ultrasound inside the reactor,
**characterized in that** it further comprises:
- a heating means (10) of said reactor at a rate from 10 to 50 °C/second;
- a cooling means (35) of said reactor at a rate from 10 to 50 °C/second, said cooling means (35) being a cooling plate crossed over by a cooling circuit, said cooling means being put in touch with the reactor by means of a press.

2. Sample preparation device (1) according to claim 1, wherein the displacement fluid is an inert gas.

3. Sample preparation device (1) according to claim 1 or 2, further comprising a heating means (26) of the displacement fluid.

4. Sample preparation device (1) according to any one of claims 1 to 3 further comprising a means (32) for creating vacuum or applying pressure in the reactor.

5. Sample preparation device (1) according to any one of claims 1 to 4, further comprising an aspiration means (32) of fluids or volatilized organic molecules.

6. Sample preparation device (1) according to any one of claims 1 to 5, further comprising at least one means (32, 33) for cleaning the reactor.

7. Sample preparation device (1) according to one of claims 1 to 6, further comprising at least one retention means of the solid sample in the reactor adapted to hold the sample during the circulation of the displacement fluid.

8. Sample preparation device (1) according to one of claims 1 to 7, further comprising at least one sealed means (17, 18) for introducing a liquid substance into the reactor.

9. Sample preparation device (1) according to claim 8, further comprising at least one tank intended for containing a liquid substance, connected to said sealed means for introducing a liquid substance.

10. Sample preparation device (1) according to any one of claims 1 to 9, wherein the control means of the temperature is a temperature sensor (28) positioned in the reactor.

11. Sample preparation device (1) according to any one of claims 1 to 10, wherein the control means comprises of the pressure comprises a pressure sensor positioned in the pressure reactor.

12. Sample preparation device (1) according to any one of claims 1 to 11, wherein the reactor is made of a material selected from the group consisting of stainless steel, copper, titanium, steel or ceramic.

13. Sample preparation device (1) according to any one of claims 1 to 12, wherein the volume of the enclosure reactor is from 2 to 10 mL.

14. Analysis system **characterized in that** it comprises at least one sample preparation device (1) according to any one of claims 1 to 13, and at least an analytical instrument (22) connected downstream of said sample preparation device (1).

15. An analysis system according to claim 14, wherein the analytical instrument is a gas chromatograph (22).

16. Analysis system according to claim 14 or 15, wherein the analytical instrument (22) is a gas chromatograph coupled to a mass spectrometer (23) or to a detector.

17. Analysis system according to any one of claims 14 to 16, further comprising a scanning circuit of organic molecules comprising:
- a tank (24) of displacement fluid;
- a pipe (25) connecting said tank (24) of the displacement fluid to the sealed inlet (13) of the sample preparation device (1), and
- a pipe (30) connecting the outlet (14) of the sample preparation device (1) to the analysis instrument (22).
